# FASCICULE DE BREVET EUROPEEN

(11) **EP 1 622 581 B1**
(45) Date de publication et mention de la délivrance du brevet: **28.03.2007**
(21) Numéro de dépôt: 04758928.8
(22) Date de dépôt: 01.04.2004
(51) Int. Cl.: A61K 8/49, A61Q 5/06, A61Q 5/08

(54) **PROCEDE DE COLORATION POUR MATIERES KERATINIQUES HUMAINES AVEC EFFET ECLAIRCISSANT, COMPOSE FLUORESCENT PARTICULIER ET COMPOSITION LE COMPRENANT**
FÄRBEMETHODE FÜR MENSCHLICHE KERATINÖSE FASERN MIT AUFHELLUNGSWIRKUNG, INSBESONDERE FLUORESZENTE VERBINDUNG UND DIESE ENTHALTENDE ZUSAMMENSETZUNG
DYEING METHOD FOR HUMAN KERATINOUS FIBERS WITH LIGHTENING EFFECT, PARTICULAR FLUORESCENT COMPOUND AND COMPOSITION COMPRISING SAME

(30) Priorité: 01.04.2003 FR 0304022
(43) Date de publication de la demande: 08.02.2006
(73) Titulaire: L'ORÉAL, 75008 Paris (FR)
(72) Inventeur: GREAVES, Andrew, F-77144 Montevrain (FR); DAUBRESSE, Nicolas, F-78170 La Celles St Cloud (FR); RADISSON, Xavier, F-75014 Paris (FR)
(74) Mandataire: Wattremez, Catherine
(86) Numéro de dépôt international: PCT/FR2004/000819
(87) Numéro de publication internationale: WO 2004/091473

(56) Documents cités:
- FR-A- 2 103 210
- FR-A- 2 411 219

## Description

la présente invention a pour objet un procédé de coloration avec effet éclaircissant de matières kératiniques humaines mettant en oeuvre une composition comprenant, dans un milieu cosmétiquement acceptable, au moins un composé fluorescent particulier. Elle a de même pour objet des composés fluorescents ou non particuliers ainsi que les compositions les comprenant.

Il est fréquent que les personnes ayant une peau colorée, voire foncée, désirent s'éclaircir la peau et utilisent dans ce but des compositions cosmétiques ou dermatologiques contenant des agents de blanchiment.
Les substances les plus utilisées comme agent de blanchiment sont l'hydroquinone et ses dérivés, l'acide kojique et ses dérivés, l'acide azélaïque, l'arbutine et ses dérivés, seuls ou en association avec d'autres actifs.
Toutefois ces agents ne sont pas dépourvus d'inconvénients. En particulier, il est nécessaire de les utiliser de façon prolongée et en des quantités élevées, pour obtenir un effet de blanchiment de la peau. De plus, on n'observe pas un effet immédiat à l'application de compositions les comprenant.
Quant à l'hydroquinone et ses dérivés, ils sont connus pour leur cytotoxicité vis-à-vis du mélanocyte.
Enfin, l'acide kojique et ses dérivés présentent l'inconvénient d'être coûteux et de ne pouvoir, pour cette raison, être utilisés en quantité importante dans des produits à large diffusion commerciale.

Il subsiste donc le besoin de compositions cosmétiques permettant d'obtenir un teint plus clair, uniforme, homogène, d'aspect naturel.

Dans le domaine capillaire, pour obtenir une coloration plus claire, on met classiquement en oeuvre un procédé de décoloration chimique. Ce procédé consiste à décolorer les mélanines de ces fibres par un système oxydant, généralement constitué par du peroxyde d'hydrogène associé ou non à des persels. Cette opération peut ou non être réalisée en présence de colorants directs et/ou de colorants d'oxydation.
Ce système de décoloration présente l'inconvénient de dégrader les fibres et d'altérer leurs propriétés cosmétiques. Les cheveux ont en effet tendance à devenir rêches, plus difficilement démêlables et plus fragiles.

Il est donc souhaitable de pouvoir disposer de compositions qui permettent d'éclaircir tout en colorant les cheveux et autres fibres kératiniques humaines, de manière esthétique, sans dégrader ces fibres.

La présente invention a donc pour objet de proposer un procédé de coloration avec effet éclaircissant de matières kératiniques humaines, ainsi que des composés fluorescents particuliers et des compositions les comprenant, ne posant pas les inconvénients mentionnés ci-dessus.

Ainsi, un premier objet de l'invention est constitué par un procédé de coloration avec un effet éclaircissant, de matières kératiniques humaines, dans lequel on applique sur lesdites matières, une composition comprenant dans un milieu cosmétiquement acceptable, au moins un composé fluorescent de formule suivante : 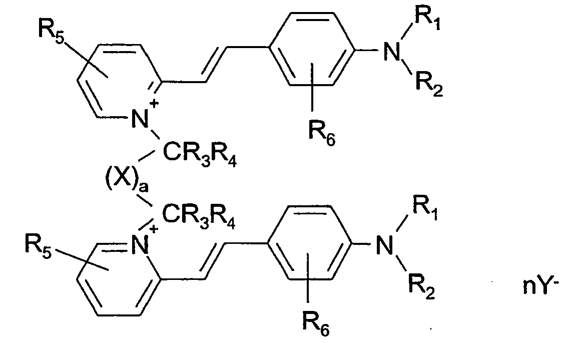 dans laquelle :
R₁, R₂, identiques ou différents, représentent :
   - un atome d'hydrogène ;
   - un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène;
   - un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
   - R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
   - R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène;
X représente :
   - un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
   - un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
   - un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
   - un radical dicarbonyle ;
   - le groupement X pouvant porter une ou plusieurs charges cationiques ;
a étant égal à 0 ou 1 ;
Y⁻, identiques ou non, représentant un anion organique ou minéral;
n étant un nombre entier au moins égal à 2 et au plus égal au nombre de charges cationiques présentes dans le composé fluorescent.

Elle a de même pour objet des composés fluorescents ou non, de formule suivante : 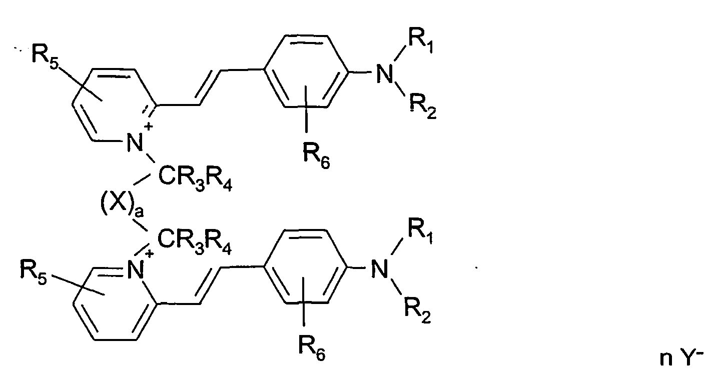 dans laquelle :
R₁, R₂, identiques ou différents, représentent :
   - un atome d'hydrogène ;
   - un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par un atome d'halogène ;
   - un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
   - R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par un atome d'halogène ;
   - R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par un atome d'halogène ;
X représente :
   - un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène;
   - un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
   - un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
   - un radical dicarbonyle ;
   - le groupement X pouvant porter une ou plusieurs charges cationiques ;
a étant égal à 0 ou 1.
Y⁻, identiques ou différents, étant un anion organique ou minéral.
n étant un nombre entier au moins égal à 2 et au plus égal au nombre de charges cationiques présentes dans le composé fluorescent ;
à l'exception des composés pour lesquels :
   - le groupement X représente un radical alkyle linéaire non substitué comprenant 1 ou 4 atomes de carbone avec a égal à 1 ou a est égal à 0 ; et les radicaux R₁ et R₂ représentent simultanément un radical méthyle ; R₅ et R₆ représentent un atome d'hydrogène ; R3 et R4 identiques représentant un atome d'hydrogène.
   - le groupement X représente un radical alkyle non substitué linéaire en C₂, un radical alkyle non substitué linéaire ou ramifié en C₃ ; a est égal à 1 ; R3 et R4 identiques représentant un atome d'hydrogène ; R₅ représente un atome d'hydrogène ; les radicaux R₁ et R₂ :
      - soit identiques, représentent un radical méthyle, R₆ représente un atome d'hydrogène, ou un radical méthyle en position ortho par rapport à l'atome de carbone du cycle benzénique portant la liaison insaturée carbone-carbone ; ou R₁ et R₂ identiques, représentent un radical éthyle, R₆ représente un atome d'hydrogène, ou un radical méthoxy en position ortho par rapport à l'atome de carbone du cycle benzénique portant la liaison insaturée carbone-carbone ;
      - soit différents, représentent un radical éthyle et un radical éthyle substitué par un groupement diméthylamino, triméthylammonium, benzyldiméthyl ammonium ;
   - le groupement X représente un radical phényle relié aux groupements CR₃R₄ par des liaisons en position 1,4 l'une par rapport à l'autre ; R₃ et R₄, identiques, représentent un atome d'hydrogène ; a est égal à 1 ; R₅ représente un atome d'hydrogène ; les radicaux R₁ et R₂ :
      - soit identiques, représentent un radical méthyle, R₆ représente un atome d'hydrogène, ou un radical méthyle en position ortho par rapport à l'atome de carbone du cycle benzénique portant la liaison insaturée carbone-carbone ; ou R₁ et R₂ identiques, représentent un radical éthyle, R₆ représente un atome d'hydrogène, ou un radical méthoxy en position ortho par rapport à l'atome de carbone du cycle benzénique portant la liaison insaturée carbone-carbone ;
      - soit différents, représentent un radical éthyle et un radical éthyle substitué par un groupement diméthylamino, triméthylammonium, benzyldiméthyl ammonium.

Elle a enfin pour objet un dispositif à plusieurs compartiments, comprenant au moins un compartiment renfermant une composition comprenant au moins un composé fluorescent ou non particulier et éventuellement au moins un colorant direct additionnel et/ou au moins une base d'oxydation et/ou au moins un coupleur, dans un milieu cosmétiquement acceptable, et au moins un autre compartiment renfermant une composition comprenant au moins un agent oxydant.

La présente invention permet de colorer tout en les éclaircissant, des matières kératiniques humaines, sans altération de ces dernières. En effet, contrairement aux procédés classiques dans lesquels on souhaite colorer tout en éclaircissant les matières kératiniques, il est nécessaire de mettre en oeuvre des composés qui peuvent à la longue créer des dommages aux dites matières (acide kojique, hydroquinone, agent oxydant).

Plus particulièrement, le procédé selon l'invention permet d'obtenir une coloration ou une teinte pour laquelle la réflectance des matières traitées conformément à l'invention, mesurée entre 550 et 700 nm, est supérieure à la réflectance des matières non traitées.

L'invention permet d'obtenir une coloration plus claire que la coloration naturelle, avec un effet esthétique satisfaisant.

Enfin, dans le cas de compositions appliquées sur des fibres kératiniques, comme notamment les cheveux, les composés mis en oeuvre présentent une bonne affinité tinctoriale pour ces fibres, de bonnes propriétés de ténacité vis-à-vis des agents extérieurs.

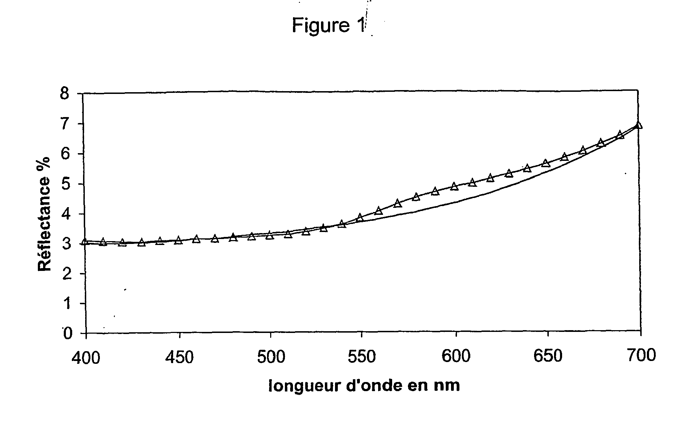

Mais d'autres caractéristiques et avantages de la présente invention apparaîtront plus clairement à la lecture de la description, des exemples et de la figure annexée représentant la réflectance en fonction de la longueur d'onde de cheveux traités avec la composition selon l'invention et de cheveux non traités, qui vont suivre.

A moins d'une indication différente, les bornes des gammes de valeurs qui sont données dans la description, sont incluses dans ces gammes.

Tout d'abord, les matières kératiniques traitées conformément au procédé selon l'invention sont d'origine humaine. Dans ce qui va suivre, il sera fait mention de matières kératiniques sachant qu'il s'agit de matières kératiniques humaines.
En outre, elles peuvent se trouver sous la forme de fibres ou non. Ainsi, lesdites matières kératiniques peuvent être la peau, les cheveux, les cils, les sourcils, la barbe, la moustache.

Selon un premier mode de réalisation de l'invention, la matière kératinique traitée est la peau. Plus particulièrement, celle-ci présente une luminance L* dans le système C.I.E. L*a*b*, mesurée au moyen d'un Colorimètre Minolta CM 2002, inférieure ou égale à 55, II est rappelé qu'une valeur de 0 pour L* équivaut au noir et 100 au blanc.
Les types de peau correspondant à cette luminance sont la peau asiatique; la peau africaine, la peau afro-américaine, la peau hispano-américaine, la peau indienne et la peau maghrébine.

Selon un deuxième mode de réalisation de l'invention, les matières kératiniques traitées se trouvent sous la forme de fibres, et plus particulièrement de fibres kératiniques pigmentées ou de fibres colorées artificiellement. De préférence, ces fibres sont des cheveux.
Avantageusement, les cheveux pigmentés ou colorés artificiellement présentent une hauteur de ton inférieure ou égale à 6 (blond foncé) et de préférence inférieure ou égale à 4 (châtain).
Rappelons que la notion de "ton" repose sur la classification des nuances naturelles, un ton séparant chaque nuance de celle qui la suit ou la précède immédiatement. Cette définition et la classification des nuances naturelles est bien connue des professionnels de la coiffure et publiée dans l'ouvrage « Sciences des traitements capillaires » de Charles ZVIAK 1988, Ed.Masson, pp.215 et 278.

Ainsi que cela a été précisé auparavant, le procédé selon l'invention consiste à mettre en oeuvre une composition comprenant au moins un composé fluorescent particulier.

Pour des raisons de clarté de l'exposé, la composition et ses divers ingrédients vont tout d'abord être décrits.

Le composé fluorescent présent dans la composition mise en oeuvre dans l'invention correspond donc à la formule générale suivante :

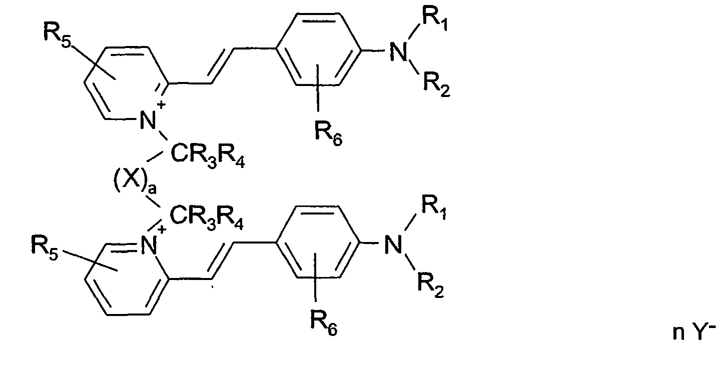

Dans cette formule, R₁, R₂, identiques ou différents, représentent :
- un atome d'hydrogène ;
- un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
- un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaire ou ramifié comprenant 1 à 4 atomes de carbone éventuellement interrompu et/ou substitué par au moins un hétéroatome et/ou groupement comprenant au moins un hétéroatome et/ou substitué par au moins un atome d'halogène ;
- R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
- R₁ ou R₂ peut éventuellement être engagé dans un hétérocycle comprenant l'atome d'azote et l'un des atomes de carbone du groupement phényle portant ledit atome d'azote.

Rappelons que les termes hétéroatomes, représentent un atome d'oxygène ou d'azote.
Parmi les groupements porteurs de tels atomes, on peut citer entre autres les groupements hydroxyle, alcoxy, carbonyle, amino, ammonium, amido (-N-CO-), carboxyle (-O-CO- ou -CO-O-).
En ce qui concerne les groupements alcényles, ces derniers comprennent une ou plusieurs liaisons carbone-carbone insaturée (-C=C-), et de préférence une seule double liaison carbone-carbone.

Dans cette formule générale, les radicaux R₁ et R₂, identiques ou non, représentent plus particulièrement :
- un atome d'hydrogène ;
- un radical alkyle comprenant 1 à 10 atomes de carbone, notamment 1 à 6 atomes de carbone, de préférence 1 à 4 atomes de carbone, éventuellement interrompu par un atome d'oxygène ou éventuellement substitué par au moins un radical hydroxyle, amino, ammonium, d'un atome de chlore ou de fluor ;
- un radical benzyle, phényle, éventuellement substitué par un radical alkyle ou alcoxy comprenant 1 à 4 atomes de carbone, de préférence 1 ou 2 atomes de carbone;
- avec l'atome d'azote, un radical hétérocylique du type pyrrolo, pyrrolidino, imidazolino, imidazolo, imidazolium, pyrazolino, pipérazino, morpholino, morpholo, pyrazolo, triazolo, éventuellement substitué par au moins un radical alkyle linéaire ou ramifié comprenant 1 à 4 atomes de carbone éventuellement interrompu et/ou substitué par un atome d'azote et/ou d'oxygène et/ou groupement portant un atome d'azote et/ou d'oxygène.

En ce qui concerne les radicaux amino ou ammonium précités, les radicaux portés par l'atome d'azote peuvent ou non être identiques et représenter plus particulièrement un atome d'hydrogène, un radical alkyle en C₁-C₁₀, de préférence en C₁-C₄, un radical arylalkyle dans lequel, plus spécialement, le radical aryle comprend 6 atomes de carbone et le radical alkyle 1 à 10 atomes de carbone, de préférence 1 à 4 atomes de carbone.

Selon un mode de réalisation avantageux de l'invention, les radicaux R₁ et R₂, identiques ou non, représentent un atome d'hydrogène ; un radical alkyle linéaire ou ramifié en C₁-C₆ ; un radical alkyle en C₂-C₆ substitué par un radical hydroxyle ; un radical alkyle en C₂-C₆ portant un groupement amino ou ammonium ; un radical chloroalkyle en C₂-C₆ ; un radical alkyle C₂-C₆ interrompu par un atome d'oxygène ou groupement en portant un (par exemple ester) ; un radical aromatique comme les phényle, benzyle, 4-méthylphényle ; un radical hétérocyclique tel que les radicaux pyrrolo, pyrrolidino, imidazolo, imidazolino, imidazolium, pipérazino, morpholo, morphollino, pyrazolo, triazolo, éventuellement substitué par au moins un radical alkyle en C₁-C₆ ou aromatique.

De préférence, les radicaux R₁ et R₂, identiques ou différents, représentent un atome d'hydrogène, un radical alkyle linéaire ou ramifié en C₁-C₆ tels que les radicaux méthyle, éthyle, n-butyle, n-propyle ; le 2-hydroxyéthyle ; un radical alkyltriméthylammonium ou alkyltriéthylammonium, le radical alkyle étant linéaire en C₂-C₆ ; un radical (di)alkylméthylamino ou (di)alkyléthylamino, le radical alkyle étant linéaire en C₂-C₆ ; -CH₂CH₂Cl ; -(CH₂)ₙ-OCH₃ ou -(CH₂)ₙ-OCH₂CH₃ avec n nombre entier variant de 2 à 6 ; -CH₂CH₂-OCOCH₃ ; -CH₂CH₂COOCH₃.
De préférence les radicaux R₁ et R₂, identiques ou non, et de préférence identiques, représentent un radical méthyle, un radical éthyle.

Les radicaux R₁ et R₂, identiques ou différents, peuvent aussi représenter un radical hétérocyclique du type pyrrolidino, 3-amino pyrrolidino, 3-(diméthyl)amino pyrrolidino, 3-(triméthyl)amino pyrrolidino, 2,5-diméthylpyrrolo, le 1H-imidazole, 4-méthyl pipérazino, 4-benzyl pipérazino, morpholo, 3,5-(ter-butyl)-1H-pyrazolo, 1H-pyrazolo, 1 H-1,2,4-triazolo.

Les radicaux R₁ et R₂, identiques ou différents, peuvent aussi représenter être reliés de manière à former un hétérocycle de formules (I) et (II) suivantes : 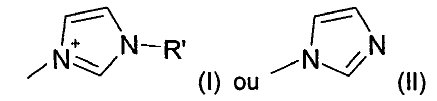 dans lesquelles R' représente un atome d'hydrogène, un radical alkyle en C₁-C₃, -CH₂CH₂OH, -CH₂CH₂OCH₃.

Conformément à un mode de réalisation plus particulier de l'invention, R₅, identiques ou non, représentent un atome d'hydrogène, un atome de fluor ou de chlore, un radical alkyle linéaire ou ramifié comprenant 1 à 4 atomes de carbone éventuellement interrompu par un atome d 'oxygène ou d'azote.
Il est précisé que le substituant R₅, s'il est différent de l'hydrogène, se trouve avantageusement en position(s) 3 et/ou 5 par rapport au carbone du cycle portant l'azote substitué par les radicaux R₁ et R₂, et de préférence en position 3 par rapport à ce carbone.
Avantageusement, les radicaux R₅, identiques ou non, représentent un atome d'hydrogène ; un radical alkyle linéaire ou ramifié en C₁-C₄; -O-R₅₁ avec R₅₁ représentant un radical alkyle linéaire en C₁-C₄ ; -R₅₂-O-CH₃ avec R₅₂ représentant un radical alkyle linéaire en C₂-C₃; -R₅₃ - N (R₅₄)₂ dans laquelle R₅₃ représente un radical alkyle linéaire en C₂-C₃, R₅₄, identiques ou différents, représentent un atome d'hydrogène ou un radical méthyle.
De préférence R₅, identiques ou non, représentent l'hydrogène, un méthyle, un méthoxy, et de préférence, R₅ représente un atome d'hydrogène.

Selon un mode de réalisation particulier, les radicaux R₆, identiques ou différents, représentent un atome d'hydrogène; un radical alkyle linéaire ou ramifié en C₁-C₄ ; -X avec X représentant un atome de chlore, de brome ou de fluor ; -R₆₁-O-R₆₂ avec R₆₁ représentant un radical alkyle linéaire en C₂-C₃ et R₆₂ représente le radical méthyle ; -R₆₃-N(R₆₄)₂ avec R₆₃ représentant un radical alkyle linéaire en C₂-C₃, R₆₄, identiques ou différents, représentent un atome d'hydrogène ou un radical méthyle, -N(R₆₅)₂ dans laquelle R₆₅, identiques ou différents, représentent un atome d'hydrogène, un radical alkyle linéaire en C₂-C₃ ; -NHCO R₆₆ avec R₆₆ représentant un radical alkyle en C₁-C₂, un radical chloroalkyle en C₁-C₂, un radical -R₆₇-NH₂ ou -R₆₇-NH(CH₃) ou -R₆₇-N(CH₃)₂ ou -R₆₇-N⁺(CH₃)₃ ou -R₆₇-N⁺(CH₂CH₃)₃ avec R₆₇ représentant un radical alkyle en C₁-C₂.
Il est précisé que le substituant R₆, s'il est différent de l'hydrogène, se trouve de préférence en position 2 et/ou 4 par rapport à l'atome d'azote du cycle pyridinium, et de préférence en position 4 par rapport à cet atome d'azote.

Plus particulièrement ces radicaux R₆, identiques ou non, représentent un atome d'hydrogène ou un radical méthyle ou éthyle, et de préférence, R₆ représente un atome d'hydrogène.

En ce qui concerne les radicaux R₃, R₄, ces derniers, identiques ou non, représentent avantageusement un atome d'hydrogène, un radical alkyle comprenant 1 à 4 atomes de carbone, plus spécialement un radical méthyle. De manière préférée, R₃ et R₄ représentent chacun un atome d'hydrogène.

Comme indiqué plus haut, X représente :
- un radical alkyle, linéaire ou ramifié comprenant 1 à 14 atomes de carbone, ou alcényle comprenant 2 à 14 atomes de carbone, éventuellement interrompu et/ou substitué par au moins un hétéroatome, par au moins un groupement porteur d'au moins un hétéroatome et/ou par au moins un atome d'halogène ;
- un radical hétérocyclique comprenant 5 ou 6 chaînons, éventuellement substitué par au moins un radical alkyle linéaire ou ramifié comprenant 1 à 14 atomes de carbone, par au moins un radical aminoalkyle, linéaire ou ramifié, comprenant 1 à 4 atomes de carbone, éventuellement substitué par au moins un hétéroatome; par au moins un atome d'halogène ;
- un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement comprenant au moins un hétéroatome ;
- un radical dicarbonyle.

En outre, il est indiqué que le groupement X peut porter une ou plusieurs charges cationiques.

Ainsi, X peut représenter un radical alkyle, linéaire ou ramifié, comprenant 1 à 14 atomes de carbone, ou alcényle comprenant 2 à 14 atomes de carbone, et peut être substitué et/ou interrompu par un ou plusieurs atomes d'oxygène et/ou d'azote, et/ou par un ou plusieurs groupements porteurs d'au moins un hétéroatome , et/ou par un atome de fluor, de chlore.
Parmi les groupements de ce type, on peut citer tout particulièrement les groupements hydroxyle, alcoxy (avec notamment un radical R de type alkyle en C₁-C₄), amino, ammonium, amido, carbonyle, carboxyle (-COO-, -O-CO-) avec notamment un radical de type alkyloxy.
Notons que l'atome d'azote, s'il est présent, peut se trouver sous une forme quatemisée ou non. Dans ce cas, le ou les deux autres radicaux portés par l'atome d'azote quatemisé ou non, sont identiques ou non et peuvent être un atome d'hydrogène, un radical alkyle en C₁-C₄, de préférence le méthyle.

Selon une autre variante, le groupement X représente un radical hétérocyclique comprenant 5 ou 6 chaînons, du type imidazolo, pyrazolo, triazino, pyridino, éventuellement substitué par au moins un radical alkyle linéaire ou ramifié comprenant 1 à 14 atomes de carbone, plus particulièrement 1 à 10 atomes de carbone, de préférence de 1 à 4 atomes de carbone ; par au moins un radical aminoalkyle, linéaire ou ramifié, comprenant 1 à 10 atomes de carbone, de préférence de 1 à 4 atomes de carbone, éventuellement substitué par un groupement comprenant au moins un hétéroatome (de préférence un radical hydroxyle), ou par un atome d'halogène. A noter que le groupement amino est de préférence lié à l'hétérocycle.

Conformément à une autre possibilité, le groupement X représente un radical aromatique (comprenant de préférence 6 atomes de carbone) ou diaromatique condensé ou non (comprenant notamment de 10 à 12 atomes de carbone), séparé ou non par un radical alkyle comprenant 1 à 4 atomes de carbone, le ou les radicaux aryles étant éventuellement substitués par au moins au moins un atome d'halogène et/ou par au moins un radical alkyle comprenant 1 à 10 atomes de carbone, de préférence 1 à 4 atomes de carbone, éventuellement interrompu par au moins un atome d'oxygène et/ou d'azote, et/ou groupement comprenant au moins un hétéroatome (comme un radical carbonyle, carboxyle, amido, amino, ammonium).
Il est à noter que le radical aromatique, de préférence un radical phényle, est relié aux groupements CR₃R₄ par l'intermédiaire de liaisons en positions 1,2 ; 1,3 ou 1;4, de préférence en positions 1,3 et 1,4. Si le radical phényle relié par l'intermédiaire de liaisons en positions 1,4, porte un ou deux substituants, ce ou ces derniers sont situés de préférence en position 1,4 par rapport à l'un des groupements CR₃R₄. Si le radical phényle relié par l'intermédiaire de liaisons en positions 1,3, porte un ou deux substituants, ce ou ces derniers sont situés de préférence en position 1 et/ou 3 par rapport à l'un des groupements CR₃R₄.

Au cas où le radical est diaromatique, il est de préférence non condensé et comprend deux radicaux phényles séparés ou non par une liaison simple (soit un carbone de chacun des deux cycles) ou par un radical alkyle, de préférence de type CH₂ ou C(CH₃)₂. De manière préférée, les radicaux aromatiques ne portent pas de substituant.
Il est à noter que ledit radical diaromatique est relié aux groupements CR₃R₄ par l'intermédiaire de liaisons en positions 4,4'.

A titre d'exemples de groupements X convenables, on peut citer notamment les radicaux alkyle linéaires ou ramifiés comprenant 1 à 13 atomes de carbone tels que méthylène, éthylène, propylène, isopropylène, n-butylène, pentylène, hexylène ; le 2-hydroxypropylène, le 2-hydroxy n-butylène ; les radicaux alkylènes en C₁-C₁₃, substitués
ou interrompus par un ou plusieurs atomes d'azote et/ou d'oxygène, et/ou groupements portant au moins un hétéroatome (hyroxyle, amino, ammonium, carbonyle, carboxyle, par exemple) tels que -CH₂CH₂OCH₂CH₂-, le 1,6-didéoxy-d-mannitol, -CH₂N⁺(CH₃)₂CH₂-, -CH₂CH₂N⁺(CH₃)₂-(CH₂)₆N⁺(CH₃)₂-CH₂CH₂-, CO-CO-, le 3,3-diméthylpentylène, le 2-acétoxyéthylène, le butylènel,2,3,4 tétraol ; -CH=CH- ; les radicaux aromatiques ou diaromatiques substitués par un ou plusieurs radicaux alkyle, par un ou plusieurs groupements portant au moins un hétéroatome et/ou par un ou plusieurs atomes d'halogène, tels que le 1,4-phénylène, le 1,3-phénylène, le 1,2-phénylène, le 2,6-fluorobenzène, le 4,4'-biphénylène, le 1,3-(5-méthyl benzène), le 1,2-bis(2-méthoxy)benzène, le bis(4-phényl)méthane, le 3,4 benzoate de méthyle, le 1,4-bis(amido méthyl)phényle; les radicaux de type hétérocycliques comme la pyridine, ou dérivé tel que le 2,6-bispyridine, l'imidazole, l'imidazolium, la triazine.

X représente, selon un mode de réalisation plus particulier de l'invention, un radical alkyle linéaire ou ramifié en C₁-C₁₃ ; -CH₂CH(OH)CH₂- ; -CH₂CH(Cl)CH₂- ; -CH₂CH₂-OCOCH₂- ; -CH₂CH₂COOCH₂- ; -Ra-O- Rb- avec Ra représentant un radical alkyle linéaire en C₂-C₆ et Rb représente un radical alkyle linéaire en C₁-C₂ ; -Rc-N(Rd)-Re- avec Rc représentant un radical alkyle en C₂-C₉, Rd représentant un atome d'hydrogène, un radical alkyle en C₁-C₂ et Re représentant un radical alkyle en C₁-C₆ ; -Rf-N⁺(Rg)₂-Rh- avec Rf représentant un radical alkyle linéaire en C₂-C₉, Rg, de préférence identiques, représentent un radical alkyle en C₁-C₂, Rh représente un radical alkyle linéaire en C₁-C₆ ; -CO-CO-.

X peut de plus représenter un radical imidazole, éventuellement substitué par au moins un radical alkyle comprenant 1 à 14 atomes de carbone, plus particulièrement 1 à 10 atomes de carbone, de préférence de 1 à 4, et par exemple les radicaux divalents de formule suivante : 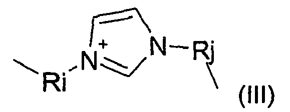 dans laquelle Ri et Rj, identiques ou non, représentent un radical alkyle linéaire en C₁-C₆ ;

X peut de même être choisi parmi les radicaux divalents dérivés de triazine suivants :

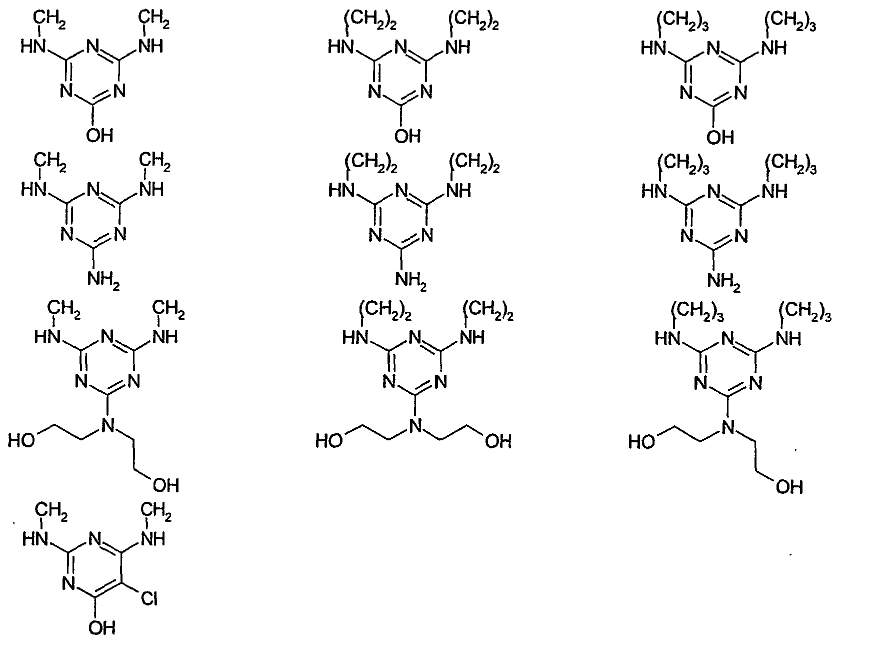

Selon une autre possibilité, X peut représenter les radicaux divalents aromatiques suivants :

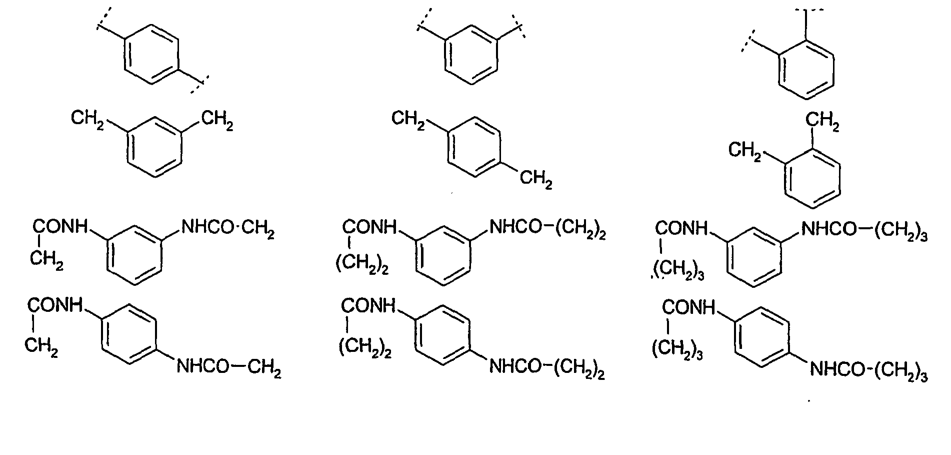

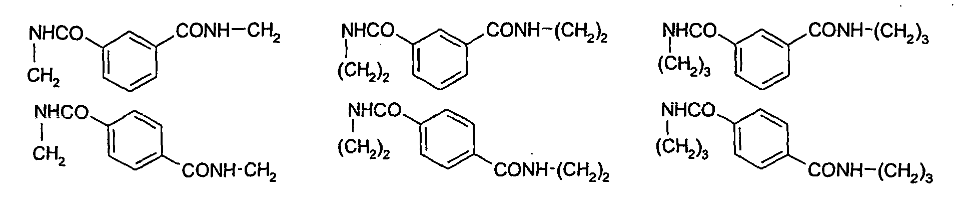

Dans la formule générale de ces composés fluorescents, Y⁻ représente un anion organique ou minéral. S'il y a plusieurs anions Y⁻, ces derniers peuvent ou non être identiques.

Parmi les anions d'origine minérale, on peut citer sans intention de s'y limiter les anions provenant d'atomes d'halogène, tels que les chlorures de préférence, les iodures, les sulfates ou bisulfates, les nitrates, les phosphates, les hydrogénophosphates, les dihydrogénophosphates, les carbonate, les bicarbonates.
Parmi les anions d'origine organique, on peut citer les anions provenant des sels d'acides mono- ou polycarboxyliques, sulfoniques, sulfuriques, saturés ou non, aromatiques ou non, éventuellement substitués par au moins un radical hydroxyle, amino, ou atomes d'halogène. A titre d'exemples non limitatifs, conviennent les acétates, hydroxyactétates, aminoacétates, (tri)chloroacétates, benzoxyactétates, propionates et dérivés portant un atome de chlore, fumarates, oxalates, acrylates, malonates, succinates, lactates, tartrates, glycollates citrates, les benzoates et dérivés portant un radical méthyle ou amino, les alkylsulfates, les tosylates, les benzènesulfonates, toluènesulfonates, etc.
De préférence, le ou les anions Y, identiques ou non, sont choisis parmi le chlore, le sulfate, le méthosulfate, l'éthosulfate.

Enfin, le nombre n, entier, est au moins égal à 2 et au plus égal au nombre de charges cationiques présentes dans le composé fluorescent.

De préférence les composés fluorescents qui viennent d'être détaillés sont des composés symétriques.

Ces composés peuvent être synthétisés en mettant en faisant réagir dans une première étape de α-picoline avec un réactif comprenant deux groupes partant qui peuvent être choisis parmi les atomes d'halogène, de préférence le brome, éventuellement le chlore,
ou les groupements de type tolylsulfonyle ou méthylesulfonyle.
Cette première étape peut avoir lieu en présence d'un solvant, bien qu'il ne soit pas obligatoire, comme par exemple le diméthylformamide.
Le nombre de moles d'α-picoline est en général voisin de 2 pour une mole de réactif comprenant les groupes partant.
En outre, la réaction est habituellement mise en oeuvre au reflux du réactif et/ou du solvant s'il est présent.

Le produit issu de cette première étape est ensuite contacté avec un aldéhyde correspondant de formule suivante : 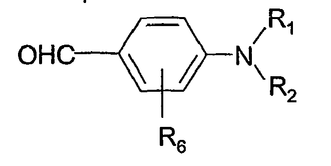 dans laquelle R₁, R₂ et R₆ ont les mêmes significations que précédemment indiquées.
Là encore, la réaction peut être effectuée en présence d'un solvant approprié, de préférence au reflux.
Il est à noter que les radicaux R₁ et R₂ de l'aldéhyde peuvent avoir la signification indiquée dans la formule générale détaillée auparavant.
Il est aussi possible de mettre en oeuvre un aldéhyde pour lequel lesdits radicaux représentent des atomes d'hydrogène et effectuer conformément à des méthodes classiques, la substitution de ces atomes d'hydrogène par des radicaux appropriés tels que décrits dans la formule générale une fois la deuxième étape terminée.

On pourra notamment se référer à des synthèses telles que décrites dans US 4256458.

Notons que dans cette référence, les composés ne sont pas décrits comme applicables à la coloration de matières kératiniques humaines, et encore moins à la coloration avec un effet éclaircissant sans avoir la nécessiter de recourir à un composé oxydant spécifique.
En effet, tous les composés décrits sont appliqués pour la coloration dans l'industrie papetière. Or il n'est pas envisageable de transposer l'enseignement obtenu à partir de résultats de coloration de composés cellulosiques à un effet dans la coloration de matières kératiniques humaines, du fait que les natures et compositions de ces deux matières sont totalement dissemblables.
En outre, ce document ne mentionne pas un quelconque effet de fluorescence pour certains des composés décrits, ni l'usage que l'on pourrait en faire dans la coloration de matières kératiniques humaines et encore moins les résultats surprenants que l'on obtiendrait grâce à leur mise en oeuvre.

Il est à noter que les composés fluorescents qui viennent d'être décrits sont des composés qui absorbent la lumière dans la partie visible du spectre et éventuellement dans la zone de l'ultraviolet, et ré-émettent une lumière fluorescente dans le spectre du visible, de plus grande longueur d'onde que celle de la lumière absorbée. De manière appropriée, la longueur d'onde de la lumière ré-émise est comprise entre 500 et 650 nm.

Conformément à l'invention, le ou les composés fluorescents peuvent se trouver sous une forme soluble ou non dans le milieu de la composition, à température ambiante (entre 15 et 25°C).

De préférence, le composé fluorescent est choisi parmi les composés solubles dans le milieu de la composition.

Selon un mode de réalisation particulier du procédé de l'invention, la solubilité du composé fluorescent dans le milieu de la composition est d'au moins 0,001g/l, plus particulièrement d'au moins 0,5g/l, de préférence d'au moins 1 g/l, et de manière encore plus préférée d'au moins 5 g/l, à une température comprise entre 15 et 25°C.

Il n'est pas exclu de mettre en oeuvre de tels composés avec des composés fluorescents additionnels solubles dans le milieu.

A titre d'exemples de famille de composés convenables, on peut citer les composés fluorescents appartenant aux familles suivantes: les naphtalimides ; les coumarines cationiques ou non ; les xanthénodiquinolizines (comme notamment les sulforhodamines) ; les azaxanthènes ; les naphtolactames ; les azlactones ; les oxazines ; les thiazines ; les dioxazines, les pyrènes, les nitrobenzoxadiazoles, seuls ou en mélanges.

A titre d'exemples plus particulier, on peut citer notamment:
- les composés de structure suivante : 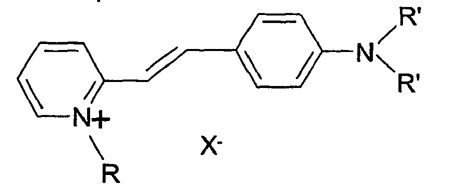 formule dans laquelle R représente un radical méthyle ou éthyle; R' représente un radical méthyle, X- un anion du type iodure, sulfate, méthosulfate.
   A titre d'exemple de composé de ce type on peut citer le Photosensitiving Dye NK-557 commercialisé par la société Ubichem, pour lequel R représente un radical éthyle, R' un radical méthyle et X- un iodure.
- le Jaune Brilliant B6GL commercialisé par la société SANDOZ et de structure suivante : 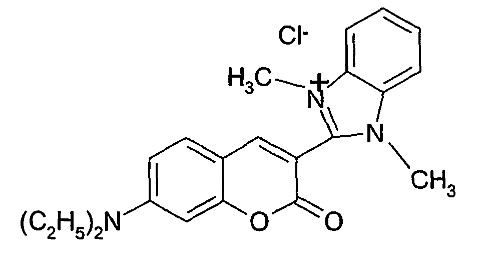
- le Basic Yellow 2, ou Auramine O commercialisé par les sociétés Prolabo, Aldrich ou Carlo Erba et de structure suivante : 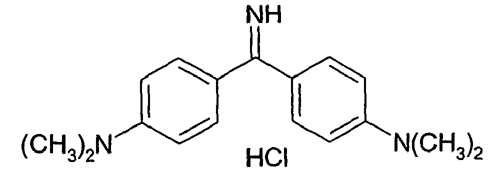 monochlorhydrate de 4,4'-(imidocarbonyl)bis(N,N-diméthylaniline) ; numéro CAS 2465-27-2.

Par ailleurs, ils peuvent aussi être mis en oeuvre avec des composés fluorescents additionnels non solubles dans le milieu, parmi lesquels on peut citer les composés à base d'oxyde de zinc, de sulfure de zinc, ainsi que des composés fluorescents organiques fabriqués à partir de colorants fluorescents qui sont préalablement dissous dans une résine support afin d'obtenir un solide qui est ensuite broyé.

Conformément à un mode de réalisation particulier de l'invention, la quantité en composé fluorescent représente 0,01 à 20% en poids par rapport au poids total de la composition, plus particulièrement 0,05 à 10% en poids par rapport à cette référence, de préférence de 0,1 à 5% en poids par rapport à cette référence.

Quant à la teneur en fluorescent additionnel, s'il(s) est(sont) présent(s), celle-ci représente plus particulièrement 0,05 à 10% en poids par rapport au poids total de la composition, de préférence 0,1 à 5% en poids par rapport à la même référence.

Le milieu cosmétiquement acceptable est généralement constitué par de l'eau ou par un mélange d'eau et d'au moins un solvant organique.
A titre de solvant organique, on peut par exemple citer, les alcanols, linéaires ou ramifiés, comprenant 1 à 4 atomes de carbone, tels que l'éthanol, l'isopropanol ; les polyols et éthers de polyols comme le glycérol, le 2-butoxyéthanol, le propylèneglycol, le monométhyléther de propylèneglycol, le monoéthyléther, le monométhyléther du diéthylèneglycol, le diméthoxyéthane, les alcools aromatiques comme l'alcool benzylique ou le phénoxyéthanol, les cétones comprenant 3 à 4 atomes de carbone, les acétates d'alkyle en C₁-C₄, ces composés étant seuls ou en mélanges.

A titre d'illustration, les solvants, s'ils sont présents, représentent 1 à 40 % en poids environ par rapport au poids total de la composition, et de manière plus avantageuse de 5 à 30 % en poids environ par rapport à la même référence.

Le pH de la composition mise en oeuvre dans le procédé selon l'invention est généralement compris entre 3 et 12 environ, et de préférence entre 5 et 11 environ.

Il peut être ajusté à la valeur désirée au moyen d'agents acidifiants ou alcalinisants habituellement utilisés.
Parmi les agents acidifiants, on peut citer, à titre d'exemples, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.
Parmi les agents alcalinisants on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 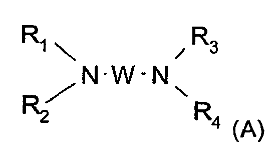 dans laquelle W est un reste propylène éventuellement substitué par un groupement hydroxyle ou un radical alkyle en C₁-C₆ R₁, R₂, R₃ et R₄, identiques ou différents, représentent un atome d'hydrogène, un radical alkyle en C₁-C₆ éventuellement porteur d'au moins un radical hydroxyle.

La composition peut de plus comprendre au moins un colorant direct additionnel non fluorescent.

Plus particulièrement, ledit colorant additionnel est de nature non ionique, cationique ou anionique.

Généralement, ces colorants directs sont choisis parmi les colorants benzéniques nitrés, les colorants azoïques, azométhiniques, méthiniques, anthraquinoniques, naphtoquinoniques, benzoquinoniques, phénotiaziniques indigoïdes, xanthéniques, phénanthridiniques, phtalocyanines, ceux dérivés du triarylméthane, seuls ou en mélanges.

Il peut par exemple être choisi parmi les colorants benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le 1-amino-3-nitro-6-hydroxybenzène.
- le 1-(β-aminoéthyl)amino-2-nitro-4-(β-hydroxyéthyloxy) benzène,
- le 1-(β,γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.

La composition mise en oeuvre dans le cadre de cette première variante peut également comprendre, en addition ou en remplacement de ces colorants benzéniques nitrés, un ou plusieurs colorants directs additionnels choisis parmi les colorants benzéniques nitrés jaunes, jaune-verts, bleus ou violets, les colorants azoïques, les colorants anthraquinoniques, naphtoquinoniques ou benzo-quinoniques, les colorants indigoïdes, ou les colorants dérivés du triarylméthane.

Ces colorants directs additionnels peuvent notamment être des colorants basiques parmi lesquels on peut citer plus particulièrement les colorants connus dans le Color Index, 3ème édition, sous les dénominations "Basic Brown 16", "Basic Brown 17", "Basic Yellow 57", "Basic Red 76", "Basic Violet 10", "Basic Blue 26" et "Basic Blue 99", ou des colorants directs acides parmi lesquels on peut plus particulièrement citer les colorants connus dans le Color Index, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore des colorants directs cationiques tels que ceux décrits dans les demandes de brevet WO 95/01772, WO 95/15144 et EP 714954 et dont le contenu fait partie intégrante de la présente invention.

Parmi les colorants directs additionnels benzéniques nitrés jaunes et jaune-verts, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- ie 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

Parmi les colorants directs additionnels benzéniques nitrés bleus ou violets, on peut par exemple citer les composés choisis parmi :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 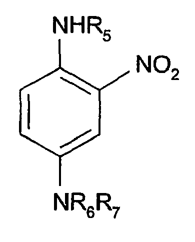
dans laquelle :
- R₆ représente un radical alkyle en C₁-C₄, un radical β-hydroxyéthyle ou β-hydroxypropyle ou γ-hydroxypropyle ;
- R₅ et R₇, identiques ou différents, représentent un radical β-hydroxyéthyle, -β-hydroxypropyle, γ-hydroxypropyle, ou β,γ-dihydroxypropyle, l'un au moins des radicaux R₆, R₇ ou R₅ représentant un radical γ-hydroxypropyle et R₆ et R₇ ne pouvant désigner simultanément un radical β-hydroxyéthyle lorsque R₆ est un radical γ-hydroxypropyle, telles que celles décrits dans le brevet français FR 2 692 572.

Lorsqu'ils sont présents, le ou les colorants directs additionnels non fluorescents représentent de préférence de 0,0005 à 12 % en poids environ du poids total de la composition, et encore plus préférentiellement de 0,005 à 6 % en poids environ de ce poids.

Toujours dans le cas où la composition est mise en oeuvre pour la coloration avec effet d'éclaircissement, de fibres kératiniques, comme les cheveux, celle-ci peut en outre comprendre au moins une base d'oxydation.

La base d'oxydation peut être choisie parmi les bases d'oxydation classiquement utilisées pour les colorations d'oxydation, comme par exemple les paraphénylène-diamines, les bis-phénylalkylènediamines, les para-aminophénols, les orthoamino-phénols et les bases hétérocycliques et leurs sels d'addition avec un acide ou avec un agent alcalin.

Parmi les paraphénylènediamines, on peut plus particulièrement citer à titre d'exemple, la paraphénylènediamine, la paratoluylènediamine, la 2-chloro para-phénylènediamine, la 2,3-diméthyl paraphénylènediamine, la 2,6-diméthyl para-phénylènediamine, la 2,6-diéthyl paraphénylènediamine, la 2,5-diméthyl para-phénylènediamine, la N,N-diméthyl paraphénylènediamine, la N,N-diéthyl para-phénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro para-phénylènediamine, la 2-isopropyl paraphénylène diamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) para-phénylène diamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy para-phénylène diamine, la N-(β-méthoxyéthyl) paraphénylènediamine et la 4'aminophényl 1-(3-hydroxy) pyrrolidine, et leurs sels d'addition avec un acide ou avec un agent alcalin.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-amino phényl) tétraméthylènediamine, la N,N'-bis-(β-hydroxyéthyl) N,N'-bis-(4-aminophényl) tétraméthylènediamine, la N,N'-bis-(4-méthylamino phényl) tétraméthylènediamine, la N,N'-bis-(éthyl) N,N'-bis-(4'-amino, 3'-méthyl phényl) éthylènediamine, le 1,8-bis-(2,5-diaminophénoxy)-3,5-dioxaoctane, et leurs sels d'addition avec un acide ou avec un agent alcalin.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxy méthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide ou avec un agent alcalin.

Parmi les ortho-aminophénols, on peut citer par exemple, le 2-amino phénol, le 2-amino 5-méthyl phénol, le 2-amino 6-méthyi phénol, le 5-acétamido 2-amino phénol, et leurs sels d'addition avec un acide ou avec un agent alcalin.

Parmi les bases hétérocycliques, on peut notamment citer les dérivés pyridiniques, les dérivés pyrimidiniques et les dérivés pyrazoliques et leurs sels d'addition avec un acide ou avec un agent alcalin.

La ou les bases d'oxydation, si elles sont présentes, représentent plus particulièrement de 0,0005 à 12 % en poids du poids total de la composition, et de préférence de 0,005 à 6 % en poids par rapport à la même référence.

Lorsqu'elle est destinée à la coloration d'oxydation de fibres kératiniques, telles que les cheveux, la composition peut également comprendre, au moins un coupleur de façon à modifier ou à enrichir en reflets les nuances obtenues en mettant en oeuvre le composé fluorescent et la base d'oxydation.

Les coupleurs utilisables peuvent être choisis parmi les coupleurs utilisés de façon classique dans ce domaine et parmi lesquels on peut notamment citer les métaphénylènediamines, les méta-aminophénols, les métadiphénols et les coupleurs hétérocycliques et leurs sels d'addition avec un acide ou avec un agent alcalin.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, et leurs sels d'addition avec un acide ou avec un agent alcalin.

Lorsqu'ils sont présents, le ou les coupleurs représentent par exemple de 0,0001 à 10 % en poids du poids total de la composition et plus spécialement de 0,005 à 5 % en poids par rapport à la même référence.

D'une manière générale, les sels d'addition avec un acide utilisables dans le cadre des compositions de l'invention (bases d'oxydation et coupleurs) sont notamment choisis parmi les chlorhydrates, les bromhydrates, les sulfates, les citrates, les succinates, les tartrates, les tosylates, les benzènesulfonates, les lactates et les acétates.
Les sels d'addition avec un agent alcalin utilisables dans le cadre de l'invention (bases d'oxydation et coupleurs) sont notamment choisis parmi les sels d'addition avec les métaux alcalins ou alcalino-terreux, avec l'ammoniaque, avec les amines organiques dont les alcanolamines et les composés de formule (I).

Lorsque la composition selon le procédé de l'invention est destinée à colorer les fibres kératiniques comme notamment les cheveux, elle peut renfermer au moins un agent oxydant, bien que ce mode de réalisation ne soit pas préféré. L'agent oxydant est choisi par exemple parmi le peroxyde d'hydrogène, le peroxyde d'urée, les bromates de métaux alcalins, les persels tels que les perborates et persulfates, et les enzymes telles que les peroxydases et les oxydo-réductases à deux ou à quatre électrons. L'utilisation du peroxyde d'hydrogène ou des enzymes est particulièrement préférée.

S'il est présent, la teneur en agent oxydant représente 0,001 et 10% en poids par rapport au poids de la composition tinctoriale prête à l'emploi.

La composition mise en oeuvre dans le procédé selon l'invention peut également comprendre divers adjuvants utilisés classiquement dans ce type de compositions, tels que des agents tensioactifs anioniques, cationiques, non ioniques, ou leurs mélanges, des polymères anioniques, cationiques, non ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des cations, des polymères cationiques ou amphotères, des silicones volatiles ou non volatiles, modifiées ou non modifiées, des agents filmogènes, des céramides, des agents conservateurs, des agents stabilisants, des agents opacifiants.

Parmi les agents épaississants, on préfère plus particulièrement utiliser les systèmes épaississants à base de polymères associatifs bien connus de l'homme de l'art et notamment de nature non ionique, anionique, cationique ou amphotère.

A noter que si la composition comprend au moins un tensioactif, la teneur est comprise entre 0,01 et 40 % en poids par rapport au poids de la composition, de préférence entre 0,1 et 30 % en poids par rapport au poids de la composition.

Bien entendu, l'homme de l'art veillera à choisir ce ou ces éventuels composés complémentaires de manière telle que les propriétés avantageuses attachées intrinsèquement à la composition ne soient pas, ou substantiellement pas, altérées par la ou les adjonctions envisagées.

La composition cosmétique mise en oeuvre pour colorer des fibres kératiniques, comme les cheveux, peut se présenter sous des formes diverses, telles que des lotions, des shampooings, des crèmes, des gels, des pâtes, ou sous toute autre forme appropriée.

Selon une variante avantageuse de l'invention, la composition selon le procédé de l'invention se trouve sous la forme d'un shampooing colorant et éclaircissant comprenant dans un milieu aqueux cosmétiquement acceptable, au moins un composé fluorescent tel que défini ci-dessus, et au moins un agent tensioactif.

De préférence, le composé fluorescent se trouvent sous une forme soluble dans le milieu.

Le ou les agents tensioactifs présents dans le shampooing peuvent être anioniques, cationiques, amphotères, ou non ioniques.
Parmi les agents tensioactifs non ioniques préférés on peut citer les alkylpolyglucosides.

Dans ces shampooings, les agents tensioactifs sont présents dans une proportion allant d'environ 4 à 30 % et de préférence d'environ 8 à 20% en poids par rapport au poids total de la composition de shampooing.

Selon une deuxième variante de l'invention, le procédé consiste à appliquer une composition sur des fibres kératiniques, notamment mais non exclusivement des cheveux, dans le but d'effectuer une coloration temporaire ou fugace avec effet d'éclaircissement, de ces fibres kératiniques, éliminable au premier shampooing ou démaquillage. Cette composition comprend de préférence au moins un composé fluorescent. Selon cette variante, le composé fluorescent se trouve sous une forme insoluble dans le milieu de la composition.

Dans le cas de cette variante, outre le composé fluorescent particulier, la composition peut comprendre au moins un pigment non fluorescent.
Les pigments non fluorescents sont habituellement choisis parmi les pigments organiques ou minéraux, cosmétiquement ou dermatologiquement acceptables.
Ils peuvent se présenter sous forme de poudre ou de pâte pigmentaire.

On pourra se reporter notamment à la demande de brevet EP 808 150 pour ce qui concerne la liste des pigments utilisables.

Si les pigments non fluorescents sont présents dans la composition, leur teneur est de préférence telle qu'elle ne masque pas l'effet de fluorescence apportée par le composé fluorescent.

A titre purement indicatif, la teneur en pigment non fluorescent, s'il est présent, est comprise entre 0,01 et 10 % en poids par rapport au poids de la composition, et de manière encore plus avantageuse, entre 0,05 et 3 % en poids par rapport à la même référence.

Le pH des compositions mises en oeuvre dans le cadre de cette deuxième variante, est plus particulièrement compris entre 6 et 8 et préférentiellement entre 6 et 7,5.

De telles compositions peuvent comprendre de plus au moins un polymère filmogène, se trouvant sous une forme soluble ou dispersée dans le milieu cosmétiquement acceptable de la composition.

Pour améliorer, si nécessaire, les propriétés du film formé, la composition peut aussi comprendre au moins un plastifiant.

Ces compositions peuvent de même contenir divers adjuvants habituellement utilisés comme par exemple les silicones insolubles ou solubles, volatiles ou non ; des protéines quaternisées ou non ; des filtres solaires ; des agents tensioactifs ; des agents antimousse ; des hydratants ; des humectants ; des émollients ; des huiles végétales ou synthétiques ; des agents conservateurs, des agents séquestrants ; des agents antioxydants ; des parfums ; des agents alcalinisants ou acidifiants, des agents de mise en suspension des pigments ; des agents épaississants.

Les compositions utilisées dans cette deuxième variante peuvent se présenter sous des formes diverses telles des liquides plus ou moins épaissis, des crèmes, des gels.
Plus spécialement, la composition peut se trouver sous la forme de mascara pour les cils
ou de mascara capillaire, à appliquer notamment au pinceau ou au peigne.

Selon une troisième variante de l'invention, le procédé consiste à appliquer sur la peau dans le but de la colorer en l'éclaircissant, une composition comprenant au moins un composé fluorescent décrit auparavant.

Selon cette variante, au moins le composé fluorescent se trouve sous une forme insoluble dans le milieu de la composition.

Dans le cas de cette variante, la composition comprend en général une phase grasse, dont une fraction n'est pas volatile (en d'autres termes ne s'évapore pas entre 15 et 25°C). Cette phase grasse peut constituer la phase continue ou la phase dispersée de la composition.

Ladite fraction non volatile peut être choisie parmi les huiles non volatiles, les cires, les gommes, les résines et/ou les corps gras pâteux d'origine animale, végétale, minérale ou synthétique, et/ou leurs mélanges.

Plus particulièrement, la fraction non volatile représente 1 à 85%, de préférence encore de 1 % à 30%, en poids, par rapport au poids total de la composition.

Les compositions selon cette variante de l'invention peuvent également comprendre au moins une charge particulière dite "soft-focus". Par charge, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. De plus, une charge "soft-focus" est une charge qui en plus donne de la transparence au teint et un effet flou. Cet effet soft-focus est lié à la réflectance spectrale de la charge.
Elles peuvent ainsi être choisies parmi la silice (par exemple micro-billes de silice SB-700 ou SB-150 de Miyoshi), le talc (par exemple Talc P3 de Nippon Talc), les composites silice / TiO₂ ou silice / oxyde de zinc, la poudre de polyéthylène, la poudre d'amidon, la poudre de nylon (par exemple Orgasol 2002 Extra D Nat Cos d'Atochem), les poudres de copolymères styrène/acrylique et/ou leurs mélanges.

De préférence, ces charges ont une taille moyenne de particules inférieure ou égale à 15 µm, de préférence, inférieure ou égale à 3 µm. De manière préférée, ces charges sont non sphériques.

Plus particulièrement, si elle est présente, la teneur en charge est comprise entre 0,1 à 20%, de préférence encore de 8% à 15%, en poids par rapport au poids total de la composition.

La composition selon cette variante peut de plus comprendre des additifs classiques dans le domaines, comme des filtres UV organiques hydrophiles ou liphophiles ou des filtres minéraux.
Leur teneur est habituellement comprise entre 0,1 et 20 %en poids par rapport au poids de la composition.

Par ailleurs, peuvent être présents dans cette composition, au moins un agent hydratant, comme par exemple l'urée ou ses dérivés, les polyols, comme par exemple la glycérine, le sorbitol ; des vésicules lipidiques émulsionnées notamment au moyen d'un tensioactif non ionique dans la composition, comme des protéines, des tocophérols, des acides aminés, l'allantoïne, etc.

Le pH de ce type de compositions est en général compris entre 6,5 et 7,5.

La composition selon cette variante peut se trouver notamment sous la forme d'une crème, d'un gel, d'un lait. Par exemple, la composition est un fond de teint.

Comme indiqué auparavant, les compositions mises en oeuvre dans le cadre des trois variantes et dont la nature des constituants et leurs proportions viennent d'être décrits, sont destinées à être appliquées sur des matières kératiniques.

Conformément à un premier mode de réalisation, la composition est appliquée sans rinçage puis on évapore ou on laisse évaporer le milieu.
Cette méthode est appropriée dans le cas où la matière kératinique est la peau ou si la composition est destinée à être appliquée sur des fibres kératiniques afin de colorer celles-ci de manière temporaire.

Selon un deuxième mode de l'invention, le procédé selon l'invention consiste à mettre en oeuvre les étapes suivantes :
a) on applique la composition sur les matières kératiniques pendant une durée suffisante pour développer la coloration et l'éclaircissement désirés,
b) on rince les matières kératiniques,
c) on lave éventuellement lesdites matières et on les rince,
d) on sèche ou on laisse sécher lesdites matières kératiniques.

Cette méthode est appropriée lorsque les matières kératiniques traitées sont des fibres comme les cheveux, la moustache, la barbe, les sourcils.

Il est précisé que la composition avec laquelle on lave éventuellement les fibres traitées à l'étape c) est plus particulièrement un shampooing.

Le temps nécessaire au développement de la coloration et à l'obtention de l'effet éclaircissant sur les fibres kératiniques est en général d'environ 5 à 60 minutes et plus particulièrement d'environ 5 à 40 minutes.

En outre, la température nécessaire au développement de la coloration et à l'obtention de l'effet éclaircissant sur les fibres kératiniques est généralement comprise entre la température ambiante (15 à 25°C) et 80°C et plus particulièrement entre 15 et 40°C.

Toujours dans le cas de ce deuxième mode de réalisation, et plus spécialement dans celui de la coloration directe ou d'oxydation, le procédé selon l'invention comporte une étape préliminaire consistant à stocker sous forme séparée, d'une part, une composition comprenant, dans un milieu approprié pour la teinture, au moins un composé fluorescent et éventuellement au moins un colorant direct additionnel et/ou au moins une base d'oxydation et/ou au moins un coupleur, et d'autre part, une composition renfermant, dans un milieu approprié pour la teinture, au moins un agent oxydant ; puis à procéder à leur mélange au moment de l'emploi avant d'appliquer ce mélange sur les fibres kératiniques pendant un temps suffisant pour développer la coloration et l'éclaircissement désirés, après quoi on rince les fibres kératiniques ; on lave éventuellement lesdites fibres kératiniques avec une composition nettoyante et on rince ; puis on sèche ou on laisse sécher les fibres kératiniques.

Un autre objet de la présente invention est constitué par des composés fluorescents ou non correspondant à la formule donnée auparavant, dans laquelle :
R₁, R₂, identiques ou différents, représentent :
   - un atome d'hydrogène ;
   - un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par un atome d'halogène ;
   - un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
   - R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par un atome d'halogène ;
   - R₁ ou R₂ peut éventuellement être engagé dans un hétérocycle comprenant l'atome d'azote et l'un des atomes de carbone du groupement phényle portant ledit atome d'azote;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par un atome d'halogène ;
X représente :
   - un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
   - un radical hétérocyclique comprenant 5 ou 6 chaînons éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène;
   - un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
   - un radical dicarbonyle ;
   - le groupement X pouvant porter une ou plusieurs charges cationiques ;
a étant égal à 0 ou 1 .
Y⁻, identiques ou différents, étant un anion organique ou minéral.
n étant un nombre entier au moins égal à 2 et au plus égal au nombre de charges cationiques présentes dans le composé fluorescent ;
à l'exception des composés pour lesquels :
- le groupement X représente un radical alkyle linéaire non substitué comprenant 1 ou 4 atomes de carbone avec a égal à 1 ou a est égal à 0 ; et les radicaux R₁ et R₂ représentent simultanément un radical méthyle ; R₅ et R₆ représentent un atome d'hydrogène ; R₃ et R₄ identiques représentant un atome d'hydrogène.
- le groupement X représente un radical alkyle non substitué linéaire en C₂, un radical alkyle non substitué linéaire ou ramifié en C₃; a est égal à 1 ; R3 et R4 identiques représentant un atome d'hydrogène ; R₅ représente un atome d'hydrogène ; les radicaux R₁ et R₂ :
   - soit identiques, représentent un radical méthyle, R₆ représente un atome d'hydrogène, ou un radical méthyle en position ortho par rapport à l'atome de carbone du cycle benzénique portant la liaison insaturée carbone-carbone ; ou R₁ et R₂ identiques, représentent un radical éthyle, R₆ représente un atome d'hydrogène, ou un radical méthoxy en position ortho par rapport à l'atome de carbone du cycle benzénique portant la liaison insaturée carbone-carbone ;
   - soit différents, représentent un radical éthyle et un radical éthyle substitué par un groupement diméthylamino, triméthylammonium, benzyldiméthyl ammonium ;
- le groupement X représente un radical phényle relié aux groupements CR₃R₄ par des liaisons en position 1,4 l'une par rapport à l'autre ; R₃ et R₄, identiques, représentent un atome d'hydrogène ; a est égal à 1 ; R₅ représente un atome d'hydrogène ; les radicaux R₁ et R₂ :
   - soit identiques, représentent un radical méthyle, R₆ représente un atome d'hydrogène, ou un radical méthyle en position ortho par rapport à l'atome de carbone du cycle benzénique portant la liaison insaturée carbone-carbone ; ou R₁ et R₂ identiques, représentent un radical éthyle, R₆ représente un atome d'hydrogène, ou un radical méthoxy en position ortho par rapport à l'atome de carbone du cycle benzénique portant la liaison insaturée carbone-carbone ;
   - soit différents, représentent un radical éthyle et un radical éthyle substitué par un groupement diméthylamino, triméthylammonium, benzyldiméthyl ammonium.

Selon une autre variante sont exclus les composés dans lesquels :
- le groupement X représente un radical alkyle linéaire comprenant 1 atome de carbone avec a égal à 1 ou a est égal à 0 ; et les radicaux R₁ et R₂ représentent un radical méthyle ; R₅ et R₆ représentent un atome d'hydrogène ;
- le groupement X représente un radical alkyle en C₂-C₃ linéaire ou ramifié ; a est égal à 1 ; les radicaux R₁ et R₂ identiques, représentent un radical méthyle ou un radical éthyle, ou différents, représentent un radical éthyle et un radical éthyle substitué par un groupement diméthylamino, triméthylammonium, benzyldiméthyl ammonium ; R₅ représente un atome d'hydrogène, R₆ représente un atome d'hydrogène, ou un radical méthyle ou méthoxy ;
- le groupement X représente un radical phényle relié aux groupements CR₃R₄ par des liaisons en position 1,4 l'une par rapport à l'autre ; a est égal à 1 ; les radicaux R₁ et R₂ identiques, représentent un radical méthyle ou un radical éthyle, ou différents, représentent un radical éthyle et un radical éthyle substitué par un groupement diméthylamino, triméthylammonium, benzyldiméthyl ammonium ; R₅ représente un atome d'hydrogène, R₆ représente un atome d'hydrogène, ou un radical méthyle ou méthoxy en position ortho par rapport à la liaison insaturée carbone - carbone.

Plus particulièrement, X représente :
- un radical alkyle ramifié comprenant 4 à 14 atomes de carbone ; un radical alkyle linéaire ou ramifié comprenant 1 à 14 atomes de carbone, substitué par un ou plusieurs atomes d'halogène et/ou hétéroatomes et/ou groupements portant un ou plusieurs hétéroatomes ; un radical alkyle linéaire ou ramifié comprenant 1 à 14 atomes de carbone interrompu par un ou plusieurs hétéroatomes et/ou groupements portant un ou plusieurs hétéroatomes ; un radical alcène, linéaire ou ramifié, comprenant 2 à 14 atomes de carbone, éventuellement interrompu et/ou substitué par un ou plusieurs hétéroatomes ou groupement portant un ou plusieurs hétéroatomes;
- un radical dicarbonyle;
- un radical hétérocyclique comprenant 5 ou 6 chaînons, du type imidazolo, pyrazolo, triazino, pyridino, éventuellement substitué par au moins un radical alkyle comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène;
- un radical aromatique en C₆ relié aux groupements CR₃R₄ par des liaisons en position 1,3 l'une par rapport à l'autre, éventuellement substitué par un ou plusieurs atomes d'halogène et/ou radical alkyle en C₁-C₄ ; lesdites liaisons comprenant un radical alkyle, identique ou non, linéaire ou ramifié en C₁-C₄ et présentant un groupement comprenant un ou plusieurs hétéroatomes directement lié ou non au radical aromatique ; un radical aromatique en C₆ relié aux groupements CR₃R₄ par des liaisons en position 1,2 l'une par rapport à l'autre, lesdites liaisons comprenant un radical alkyle, identique ou non, linéaire ou ramifié en C₁-C₄ et présentant un groupement comprenant un ou plusieurs hétéroatomes directement lié ou non au radical aromatique ; un radical aromatique en C₆ relié aux groupements CR₃R₄ par des liaisons en position 1,2 l'une par rapport à l'autre, substitué par un ou plusieurs groupements comprenant un ou plusieurs hétéroatomes ; un radical aromatique en C₆ relié aux groupements CR₃R₄ par des liaisons en position 1,4 l'une par rapport à l'autre, lesdites liaisons comprenant un radical alkyle, identique ou non, linéaire ou ramifié en C₁-C₄ et présentant un groupement comprenant un ou plusieurs hétéroatomes directement lié ou non au radical aromatique ; un radical diphényle relié aux groupements CR₃R₄ par des liaisons en position 4,4' l'une par rapport à l'autre, les deux noyaux aromatiques étant éventuellement liés au moyen d'un radical alkyle, linéaire ou ramifié en C₁-C₄;
- le groupement X pouvant porter une ou plusieurs charges cationiques.

De préférence, X représente :
- un radical alkyle ramifié comprenant 4 à 13 atomes de carbone ; un radical alkyle linéaire ou ramifié comprenant 1 à 13 atomes de carbone, substitué par au moins un atome de chlore et/ou au moins un radical hydroxyle et/ou au moins un radical acétoxy et/ou au moins un radical amino et/ou au moins un radical ammonium; un radical alkyle linéaire ou ramifié comprenant 2 à 12 atomes de carbone interrompu par un ou plusieurs atomes d'oxygène, par un ou plusieurs atomes d'azote porteurs d'un ou de deux radicaux, identiques ou non, choisis indépendamment les uns des autres, parmi les atomes d'hydrogène, les radicaux alkyle, linéaires ou ramifiés en C₁-C₄ éventuellement porteurs d'un groupement hydroxyle ; un radical alcène, linéaire ou ramifié, comprenant 2 à 12 atomes de carbone, et comprenant une liaison carbone-carbone insaturée ;
- un radical imidazole éventuellement substitué par au moins un radical alkyle en C₁-C₁₄ ;
- un radical aromatique en C₆ relié aux groupements CR₃R₄ par des liaisons en position 1,3 l'une par rapport à l'autre, éventuellement substitué par un ou plusieurs atomes de fluor et/ou méthyle ; lesdites liaisons comprenant un radical alkyle, identique ou non, linéaire ou ramifié en C₁-C₄ et présentant un groupement comprenant un ou plusieurs hétéroatomes directement lié ou non au radical aromatique ; un radical aromatique en C₆ relié aux groupements CR₃R₄ par des liaisons en position 1,4 l'une par rapport à l'autre, lesdites liaisons comprenant un radical alkyle, identique ou non, linéaire ou ramifié en C₁-C₄ et présentant un groupement amide directement lié au radical aromatique ;
- le groupement X pouvant porter une ou plusieurs charges cationiques.

Conformément à un mode de réalisation plus particulier, X représente :
- un radical alkyle ramifié comprenant 4 à 13 atomes de carbone ; un radical alkyle linéaire ou ramifié comprenant 1 à 13 atomes de carbone, substitué par au moins un atome de chlore et/ou au moins un radical hydroxyle et/ou au moins un radical acétoxy et/ou au moins un radical amino et/ou au moins un radical ammonium; un radical alkyle linéaire ou ramifié comprenant 2 à 12 atomes de carbone interrompu par un ou plusieurs atomes d'oxygène, par un ou plusieurs atomes d'azote porteurs d'un ou de deux radicaux, identiques ou non, choisis indépendamment les uns des autres, parmi les atomes d'hydrogène, les radicaux alkyle, linéaires ou ramifiés en C₁-C₄ éventuellement porteurs d'un groupement hydroxyle ; un radical alcène, linéaire ou ramifié, comprenant 2 à 12 atomes de carbone, et comprenant une liaison carbone-carbone insaturée ;
- un radical pyridine relié aux groupements CR₃R₄ par des liaisons en positions 2 et 6 l'une par rapport à l'autre ;
- un radical imidazole éventuellement substitué par au moins un radical alkyle en C₁-C₁₄ ;
- un radical aromatique en C₆ relié aux groupements CR₃R₄ par des liaisons en position 1,3 l'une par rapport à l'autre, éventuellement substitué par un ou plusieurs atomes de fluor et/ou méthyle ; lesdites liaisons comprenant un radical alkyle, identique ou non, linéaire ou ramifié en C₁-C₄ et présentant un groupement comprenant un ou plusieurs hétéroatomes directement lié ou non au radical aromatique ; un radical aromatique en C₆ relié aux groupements CR₃R₄ par des liaisons en position 1,4 l'une par rapport à l'autre, lesdites liaisons comprenant un radical alkyle, identique ou non, linéaire ou ramifié en C₁-C₄ et présentant un groupement amide directement lié au radical aromatique ;
- le groupement X pouvant porter une ou plusieurs charges cationiques.

Ce qui a été indiqué auparavant à propos de la nature des divers radicaux R₁ à R₆, des anions Y, et des exemples précis de X reste valable dans le cas présent et l'on pourra s'y référer, dès l'instant que les conditions ci-dessus sont respectées.

La présente invention a pour objet une composition comprenant, dans un milieu cosmétiquement acceptable, au moins un composé fluorescent tel qu'il vient d'être mentionné.

Avantageusement, la quantité en ce composé fluorescent dans la composition selon l'invention, est comprise entre 0,01 et 20% en poids, plus particulièrement entre 0,05 et 10% en poids, de préférence entre 0,1 et 5% en poids par rapport au poids total de la composition.

Il est à noter que la quantité de composé(s) fluorescent(s) présent(s) dans la composition, est plus particulièrement telle qu'après application sur la matière kératinique, plus particulièrement des fibres dont la hauteur de ton est d'au plus 6, de préférence d'au plus 4, la composition donne une réflectance, mesurée entre 500 et 700 nm, supérieure à la réflectance de la matière kératinique non traitée.

Plus particulièrement, le composé fluorescent est choisi parmi les composés tels que leur solubilité dans le milieu de la composition est d'au moins 0,01g/l, plus particulièrement d'au moins 0,5g/l, de préférence d'au moins 1 g/l, et de manière encore plus préférée, d'au moins 5 g/l, à une température comprise entre 15 et 25°C.

Ce qui a été indiqué auparavant concernant la description de la composition mise en oeuvre dans le procédé, et notamment sur la nature, les teneurs des divers additifs possibles reste valable et l'on pourra s'y reporter.

La composition cosmétique mise en oeuvre pour colorer les fibres kératiniques peut se présenter sous des formes diverses, telles que des lotions, des shampooings, des crèmes, des gels, des pâtes, ou sous toute autre forme appropriée.

Selon une variante avantageuse de l'invention, la composition se trouve sous la forme d'un shampooing colorant et éclaircissant comprenant dans un milieu aqueux cosmétiquement acceptable, au moins un composé fluorescent selon l'invention, et au moins un agent tensioactif.

Le ou les agents tensioactifs présents dans le shampooing peuvent être anioniques, cationiques, amphotères ou non ioniques.
Parmi les agents tensioactifs non ioniques préférés, on peut citer les alkylpolyglucosides.

Dans ces shampooings, les agents tensioactifs sont présents dans une proportion allant d'environ 4 à 30 % et de préférence d'environ 8 à 20% en poids par rapport au poids total de la composition de shampooing.

Un autre objet de l'invention est un dispositif à plusieurs compartiments, comprenant au moins un compartiment renfermant une composition comprenant au moins un composé fluorescent et éventuellement au moins un colorant fluorescent additionnel, au moins un colorant direct non fluorescent additionnel et/ou au moins une base d'oxydation et/ou au moins un coupleur, dans un milieu cosmétiquement acceptable, et au moins un autre compartiment renfermant une composition comprenant au moins un agent oxydant.

Selon un mode de réalisation particulier de dispositif de l'invention, la composition comprend au moins un composé fluorescent soluble dans le milieu.

Le dispositif à plusieurs compartiments peut être équipé d'un moyen permettant de délivrer sur les cheveux le mélange souhaité, tel que les dispositifs décrits dans FR 2 586 913.

Des exemples concrets mais non limitatifs de l'invention vont maintenant être présentés.

### EXEMPLES

### Composé fluorescent :

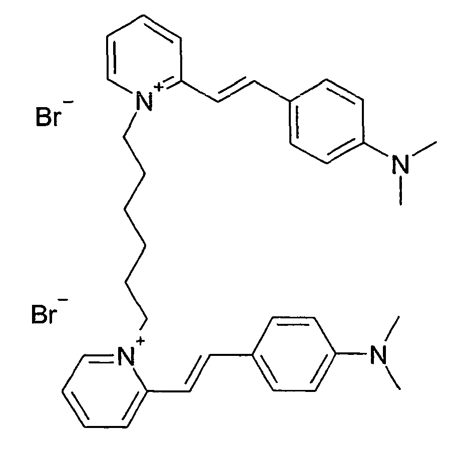

On fait réagir 93 g de 2-picoline avec 120g de 1,6 dibromohexane dans le diméthylformamide à 110°C pendant 5 heures.
On récupère le produit précipité, et on le filtre.
On solubilise 109 g du produit obtenu précédemment dans du méthanol et l'on ajoute 82,82 g de p-diméthylaminobenzaldéhyde en deux fois, en présence de pyrrolidine.
On laisse ensuite pendant 30 minutes.
On récupère le produit sous forme précipitée.

Analyse par spectroscopie de masse : 266.
Analyse élémentaire : C : 62,43 % ; H: 6,40 % ; Br : 23,07 % ; N : 8,09 %.
La formule est la suivante C₃₆H₄₄N₄.2Br.

### Coloration sur le cheveu

Le composé est solubilisé dans l'eau permutée, le pH ajusté à 7,1 avec de l'acide chlorhydrique dilué. Sa concentration dans le milieu est de 1 % en poids.
La solution colorante ainsi obtenue est appliquée sur cheveux naturels (90% blancs naturels) et châtains (hauteur de ton 4) avec un rapport de bain de 5 : 1 pendant 20 minutes à température ambiante.
Après coloration le cheveu a été rincé à l'eau et séché à température ambiante.

### Procédures de shampooing

Sur les mèches préalablement colorées, on applique 0,4g de shampooing Ultra Doux Camomille (Garnier) par gramme de cheveux selon le protocole suivant :
3 passages entre les doigts par mèche, suivis de 15 passages sous l'eau tiède et de 30 minutes de séchage. Deux et six cycles de shampooings ont été réalisés.

### Résultats des tests

Les mesures de couleur ont été effectuées à l'aide d'un spectrocolorimètre (CM3600d Minolta, composantes spéculaires inclues, illuminant D65, angle 10°).

### Montée de couleur sur cheveux naturels (90% de cheveux blancs naturels) :

| | L* | a* | b* |
|---|---|---|---|
| Cheveux naturels non teints | 57.02 | 0.71 | 13.29 |
| Cheveux naturels teints | 47.05 | 28.12 | 42.62 |

On constate que le colorant est bien monté dans le cheveu.

### Montée de couleur sur cheveux châtains

| | L* | a* | b* |
|---|---|---|---|
| Cheveux châtains non teints | 23,00 | 3,29 | 4,44 |
| Cheveux châtains teints | 23,66 | 4,78 | 5,53 |

On constate que le colorant est bien monté dans le cheveu.

### Pouvoir éclaircissant sur cheveux châtains naturels

La courbe en trait simple représente la réflectance obtenue pour les cheveux châtains non teints (témoin), la courbe marquée de triangles représente la réflectance obtenue pour des cheveux châtains traités conformément à l'invention.

On constate qu'il y a un effet d'éclaircissement des cheveux traités selon l'invention, par rapport au cheveux non teints.

## Revendications

1. Procédé de coloration avec effet éclaircissant, de matières kératiniques humaines dans lequel on applique sur lesdites matières une composition comprenant dans un milieu cosmétiquement acceptable, au moins un composé fluorescent de formule suivante : 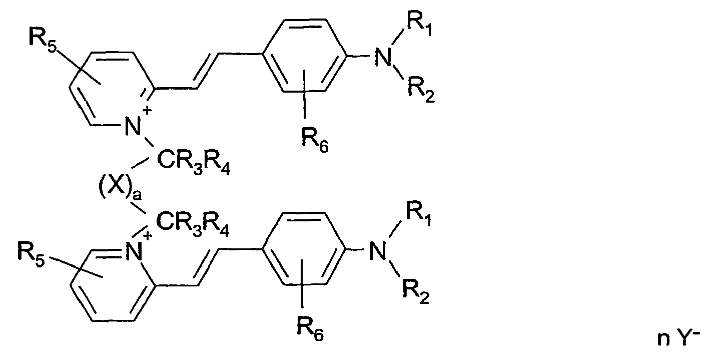 dans laquelle :
R₁, R₂, identiques ou différents, représentent :
• un atome d'hydrogène ;
• un radical alkyle, linéaire ou ramifié, comprenant 1 à 10 atomes de carbone, éventuellement interrompu et/ou substitué par au moins un hétéroatome et/ou groupement comprenant au moins un hétéroatome et/ou substitué par au moins un atome d'halogène ;
• un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
• R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
• R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
X représente :
• un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène;
• un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
• un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
• un radical dicarbonyle ;
• le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent.

2. Procédé selon la revendication précédente, **caractérisé en ce que** le composé est tel que les radicaux R₁ et R₂, identiques ou non, représentent :
• un atome d'hydrogène ;
• un radical alkyle comprenant 1 à 6 atomes de carbone, éventuellement interrompu par un atome d'oxygène ou éventuellement substitué par au moins un radical hydroxyle, amino, ammonium, d'un atome de chlore ou de fluor ;
• un radical benzyle, phényle, éventuellement substitué par un radical alkyle ou alcoxy comprenant 1 à 4 atomes de carbone, de préférence 1 ou 2 atomes de carbone ;
• avec l'atome d'azote, un radical hétérocylique du type pyrrolo, pyrrolidino, imidazolino, imidazolo, imidazolium, pyrazolino, pipérazino, morpholino, morpholo, pyrazolo, triazolo, éventuellement substitué par au moins un radical alkyle linéaire ou ramifié comprenant 1 à 4 atomes de carbone éventuellement interrompu et/ou substitué par un atome d'azote et/ou d'oxygène et/ou groupement portant un atome d'azote et/ou d'oxygène.

3. Procédé selon l'une quelconque des précédentes, **caractérisé en ce que** les radicaux R₁ et R₂, identiques ou non, et de préférence identiques, représentent un radical alkyle comprenant 1 à 4 atomes de carbone.

4. Procédé selon l'une quelconque des précédentes, **caractérisé en ce que** les radicaux R₁ et R₂, identiques ou non, et de préférence identiques, représentent un radical méthyle, un radical éthyle.

5. Procédé selon l'une quelconque des précédentes, **caractérisé en ce que** les radicaux R₅ et R₆ représentent un atome d'hydrogène.

6. Procédé selon l'une quelconque des revendications précédentes, **caractérisé en ce que** le composé est tel que le groupement X représente :
• un radical alkyle, linéaire ou ramifié, comprenant 1 à 14 atomes de carbone, ou alcényle comprenant 2 à 14 atomes de carbone, et peut être substitué et/ou interrompu par un ou plusieurs atomes d'oxygène et/ou d'azote, et/ou par un ou plusieurs groupements porteurs d'au moins un hétéroatome, comme les groupements hydroxyle, alcoxy, amino, ammonium, amido, carbonyle, carboxyle (-COO-, -O-CO-), et/ou par un atome de fluor;
• un radical dicarbonyle;
• un radical hétérocyclique comprenant 5 ou 6 chaînons, du type imidazolo, pyrazolo, triazino, pyridino, éventuellement substitué par au moins un radical alkyle linéaire ou ramifié comprenant 1 à 14 atomes de carbone, plus particulièrement 1 à 10 atomes de carbone, de préférence de 1 à 4 atomes de carbone ; par au moins un radical aminoalkyle, linéaire ou ramifié, comprenant 1 à 10 atomes de carbone, de préférence de 1 à 4 atomes de carbone, éventuellement substitué par un groupement comprenant au moins un hétéroatome, comme un radical hydroxyle, par au moins un atome d'halogène ;
• un radical aromatique comprenant 6 atomes de carbone ou diaromatique condensé ou non comprenant notamment de 10 à 12 atomes de carbone, séparé ou non par un radical alkyle comprenant 1 à 4 atomes de carbone, le ou les radicaux aryles étant éventuellement substitués par au moins au moins un atome d'halogène et/ou par au moins un radical alkyle comprenant 1 à 10 atomes de carbone éventuellement interrompu par au moins un atome d'oxygène et/ou d'azote, et/ou groupement comprenant au moins un hétéroatome, comme un radical carbonyle, carboxyle, amido, amino, ammonium.

7. Procédé selon l'une quelconque des revendications précédentes, **caractérisé en ce que** la composition comprend une quantité en composé fluorescent comprise entre 0,01 et 20% en poids, plus particulièrement entre 0,05 et 10% en poids, de préférence entre 0,1 et 5% en poids par rapport au poids total de la composition.

8. Procédé selon l'une quelconque des revendications précédentes, **caractérisé en ce que** la composition comprend au moins un composé fluorescent additionnel.

9. Procédé selon la revendication précédente, **caractérisé en ce que** la teneur en composé fluorescent additionnel est comprise entre 0,05 et 10% en poids par rapport au poids de la composition, de préférence 0,1 et 5% en poids par rapport au poids de la composition.

10. Procédé selon l'une quelconque des revendications précédentes, **caractérisé en ce que** la composition comprend au moins un colorant direct additionnel non fluorescent.

11. Procédé selon la revendication précédente, **caractérisé en ce que** le ou les colorants directs additionnels sont choisis parmi les colorants benzéniques nitrés, les colorants azoïques, azométhiniques, méthiniques, anthraquinoniques, naphtoquinoniques, benzoquinoniques, phénotiaziniques, indigoïdes, xanthéniques, phénanthridiniques, phtalocyanines, ceux dérivés du triarylméthane seuls ou en mélanges.

12. Procédé selon l'une quelconque des revendications précédentes, **caractérisé en ce que** la composition comprend une teneur en colorant direct additionnel comprise entre 0,0005 et 12 % en poids, de préférence entre 0,005 à 6 % en poids, par rapport au poids total de la composition.

13. Procédé selon l'une quelconque des revendications précédentes, **caractérisé en ce que** la composition comprend au moins un tensioactif non ionique, anionique, cationique, amphotère ou zwittérionique, ou leurs mélanges.

14. Procédé selon la revendication précédente, **caractérisé en ce que** la composition comprend une teneur en tensioactif est variant entre 0,01 et 40% en poids, de préférence entre 0,1 et 30% en poids, par rapport au poids total de la composition.

15. Procédé selon l'une quelconque des revendications précédentes, **caractérisé en ce que** le milieu cosmétiquement acceptable comprend de l'eau et éventuellement un solvant organique.

16. Procédé selon l'une quelconque des revendications précédentes, **caractérisé en ce que** la composition se trouve sous la forme d'un shampooing colorant.

17. Procédé selon l'une quelconque des revendications 1 à 15, **caractérisé en ce que** la composition se trouve sous forme d'un mascara pour les cils ou d'un mascara capillaire.

18. Procédé selon l'une quelconque des revendications 1 à 15, **caractérisé en ce que** la composition comprend au moins une base d'oxydation choisie parmi les paraphénylènediamines, les bis-phénylalkylènediamines, les para-aminophénols, les ortho-aminophénols et les bases hétérocycliques ou leurs sels d'addition avec un acide ou avec un agent alcalin.

19. Procédé selon l'une quelconque des revendications 1 à 15 et 18, **caractérisé en ce que** la composition comprend une teneur en base d'oxydation variant entre 0,0005 et 12 % en poids, de préférence entre 0,005 et 6 % en poids, par rapport au poids total de la composition.

20. Procédé selon l'une quelconque des revendications 1 à 15 et 18 ou 19, **caractérisé en ce que** la composition comprend au moins un coupleur choisi parmi les métaphénylène-diamines, les méta-aminophénols, les métadiphénols et les coupleurs hétérocycliques ou leurs sels d'addition avec un acide ou avec un agent alcalin.

21. Procédé selon l'une quelconque des revendications 1 à 15 et 18 à 20, **caractérisé en ce que** la composition comprend une teneur en coupleur variant entre 0,0001 et 10 % en poids, de préférence entre 0,005 à 5 % en poids, par rapport au poids total de la composition.

22. Procédé selon l'une quelconque des revendications 1 à 15 et 18 à 21, **caractérisé en ce que** la composition comprend au moins un agent oxydant.

23. Procédé selon la revendication précédente, **caractérisé en ce que** l'agent oxydant est choisi parmi le peroxyde d'hydrogène, le peroxyde d'urée, les bromates de métaux alcalins, les persels tels que les perborates et persulfates, et les enzymes telles que les peroxydases et les oxydo-réductases à deux ou quatre électrons.

24. Procédé selon l'une des revendications 1 à 15 et 17, **caractérisé en ce que** l'on met en oeuvre les étapes suivantes :
a) on applique la composition sur les matières kératiniques humaines,
b) on sèche ou on laisse sécher les matières.

25. Procédé selon l'une quelconque des revendications 1 à 16 et 18 à 23, **caractérisé en ce que** l'on met en oeuvre les étapes suivantes :
a) on applique la composition sur les matières kératiniques humaines, pendant un temps suffisant pour développer la coloration et l'éclaircissement désirés,
b) on rince éventuellement les matières ainsi traitées,
c) éventuellement on lave et on rince lesdites matières,
d) on sèche ou on laisse sécher les matières.

26. Composé fluorescent de formule suivante : 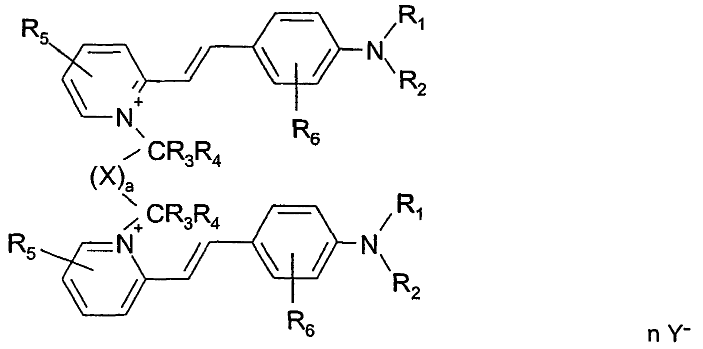 dans laquelle :
R₁, R₂, identiques ou différents, représentent :
• un atome d'hydrogène ;
• un radical alkyle, linéaire ou ramifié, comprenant 1 à 10 atomes de carbone, éventuellement interrompu et/ou substitué par au moins un hétéroatome et/ou groupement comprenant au moins un hétéroatome et/ou substitué par un atome d'halogène ;
• un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
• R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par un atome d'halogène ;
• R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par un atome d'halogène ;
X représente :
• un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
• un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
• un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
• un radical dicarbonyle ;
• le groupement X pouvant porter une ou plusieurs charges cationiques ;
a étant égal à 0 ou 1 .
Y⁻, identiques ou différents, étant un anion organique ou minéral.
n étant un nombre entier au moins égal à 2 et au plus égal au nombre de charges cationiques présentes dans le composé fluorescent;
à l'exception des composés pour lesquels :
• le groupement X représente un radical alkyle linéaire non substitué comprenant 1 ou 4 atomes de carbone avec a égal à 1 ou a est égal à 0 ; et les radicaux R₁ et R₂ représentent simultanément un radical méthyle ; R₅ et R₆ représentent un atome d'hydrogène ; R3 et R4 identiques représentant un atome d'hydrogène.
• le groupement X représente un radical alkyle non substitué linéaire en C₂, un radical alkyle non substitué linéaire ou ramifié en C₃ ; a est égal à 1 ; R3 et R4 identiques représentant un atome d'hydrogène ; R₅ représente un atome d'hydrogène ; les radicaux R₁ et R₂ :
- soit identiques, représentent un radical méthyle, R₆ représente un atome d'hydrogène, ou un radical méthyle en position ortho par rapport à l'atome de carbone du cycle benzénique portant la liaison insaturée carbone-carbone ; ou R₁ et R₂ identiques, représentent un radical éthyle, R₆ représente un atome d'hydrogène, ou un radical méthoxy en position ortho par rapport à l'atome de carbone du cycle benzénique portant la liaison insaturée carbone-carbone ;
- soit différents, représentent un radical éthyle et un radical éthyle substitué par un groupement diméthylamino, triméthylammonium, benzyldiméthyl ammonium ;
• le groupement X représente un radical phényle relié aux groupements CR₃R₄ par des liaisons en position 1,4 l'une par rapport à l'autre ; R₃ et R₄, identiques, représentent un atome d'hydrogène ; a est égal à 1 ; R₅ représente un atome d'hydrogène ; les radicaux R₁ et R₂ :
- soit identiques, représentent un radical méthyle, R₆ représente un atome d'hydrogène, ou un radical méthyle en position ortho par rapport à l'atome de carbone du cycle benzénique portant la liaison insaturée carbone-carbone ; ou R₁ et R₂ identiques, représentent un radical éthyle, R₆ représente un atome d'hydrogène, ou un radical méthoxy en position ortho par rapport à l'atome de carbone du cycle benzénique portant la liaison insaturée carbone-carbone ;
- soit différents, représentent un radical éthyle et un radical éthyle substitué par un groupement diméthylamino, triméthylammonium, benzyldiméthyl ammonium.

27. Composé selon la revendication 26, **caractérisé en ce que** les radicaux R₁ et R₂, identiques ou non, représentent :
• un atome d'hydrogène ;
• un radical alkyle comprenant 1 à 6 atomes de carbone, éventuellement interrompu par un atome d'oxygène ou éventuellement substitué par au moins un radical hydroxyle, amino, ammonium, d'un atome de chlore ou de fluor ;
• un radical benzyle, phényle, éventuellement substitué par un radical alkyle ou alcoxy comprenant 1 à 4 atomes de carbone, de préférence 1 ou 2 atomes de carbone ;
• avec l'atome d'azote, un radical hétérocylique du type pyrrolo, pyrrolidino, imidazolino, imidazolo, imidazolium, pyrazolino, pipérazino, morpholino, morpholo, pyrazolo, triazolo, éventuellement substitué par au moins un radical alkyle linéaire ou ramifié comprenant 1 à 4 atomes de carbone éventuellement interrompu et/ou substitué par un atome d'azote et/ou d'oxygène et/ou groupement portant un atome d'azote et/ou d'oxygène.

28. Composé selon l'une quelconque des revendications 26 ou 27, **caractérisé en ce que** les radicaux R₁ et R₂, identiques ou non, représentent un radical alkyle comprenant 1 à 4 atomes de carbone.

29. Composé selon la revendication précédente, **caractérisé en ce que** les radicaux R₁ et R₂, identiques ou non, représentent un radical méthyle, un radical éthyle.

30. Composé selon l'une quelconque des revendications 26 à 29, **caractérisé en ce que** les radicaux R₅ et R₆ représentent un atome d'hydrogène.

31. Composé selon l'une quelconque des revendications 26 à 30, **caractérisé en ce que** X représente :
• un radical alkyle ramifié comprenant 4 à 14 atomes de carbone ; un radical alkyle linéaire ou ramifié comprenant 1 à 14 atomes de carbone, substitué par un ou plusieurs atomes d'halogène et/ou hétéroatomes et/ou groupements portant un ou plusieurs hétéroatomes ; un radical alkyle linéaire ou ramifié comprenant 1 à 14 atomes de carbone interrompu par un ou plusieurs hétéroatomes et/ou groupements portant un ou plusieurs hétéroatomes ; un radical alcène, linéaire ou ramifié, comprenant 2 à 14 atomes de carbone, éventuellement interrompu et/ou substitué par un ou plusieurs hétéroatomes ou groupement portant un ou plusieurs hétéroatomes ;
• un radical dicarbonyle ;
• un radical hétérocyclique comprenant 5 ou 6 chaînons, éventuellement substitué par au moins un radical alkyle comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène;
• un radical aromatique en C₆ relié aux groupements CR₃R₄ par des liaisons en position 1,3 l'une par rapport à l'autre, éventuellement substitué par un ou plusieurs atomes d'halogène et/ou radical alkyle en C₁-C₄ ; lesdites liaisons comprenant un radical alkyle, identique ou non, linéaire ou ramifié en C₁-C₄ et présentant un groupement comprenant un ou plusieurs hétéroatomes directement lié ou non au radical aromatique ; un radical aromatique en C₆ relié aux groupements CR₃R₄ par des liaisons en position 1,2 l'une par rapport à l'autre, lesdites liaisons comprenant un radical alkyle, identique ou non, linéaire
ou ramifié en C₁-C₄ et présentant un groupement comprenant un ou plusieurs hétéroatomes directement lié ou non au radical aromatique ; un radical aromatique en C₆ relié aux groupements CR₃R₄ par des liaisons en position 1,2 l'une par rapport à l'autre, substitué par un ou plusieurs groupements comprenant un ou plusieurs hétéroatomes ; un radical aromatique en C₆ relié aux groupements CR₃R₄ par des liaisons en position 1,4 l'une par rapport à l'autre, lesdites liaisons comprenant un radical alkyle, identique ou non, linéaire ou ramifié en C₁-C₄ et présentant un groupement comprenant un ou plusieurs hétéroatomes directement lié ou non au radical aromatique ; un radical diphényle relié aux groupements CR₃R₄ par des liaisons en position 4,4' l'une par rapport à l'autre, les deux noyaux aromatiques étant éventuellement liés au moyen d'un radical alkyle, linéaire ou ramifié en C₁-C₄ ;
• le groupement X pouvant porter une ou plusieurs charges cationiques.

32. Composé selon l'une quelconque des revendications 26 à 31, **caractérisé en ce que** X représente :
• un radical alkyle ramifié comprenant 4 à 13 atomes de carbone ; un radical alkyle linéaire ou ramifié comprenant 1 à 13 atomes de carbone, substitué par au moins un atome de chlore et/ou au moins un radical hydroxyle et/ou au moins un radical acétoxy et/ou au moins un radical amino et/ou au moins un radical ammonium; un radical alkyle linéaire ou ramifié comprenant 2 à 12 atomes de carbone interrompu par un ou plusieurs atomes d'oxygène, par un ou plusieurs atomes d'azote porteurs d'un ou de deux radicaux, identiques ou non, choisis indépendamment les uns des autres, parmi les atomes d'hydrogène, les radicaux alkyle, linéaires ou ramifiés en C₁-C₄ éventuellement porteurs d'un groupement hydroxyle ; un radical alcène, linéaire ou ramifié, comprenant 2 à 12 atomes de carbone, et comprenant une liaison carbone-carbone insaturée;
• un radical pyridine relié aux groupements CR₃R₄ par des liaisons en positions 2 et 6 l'une par rapport à l'autre ;
• un radical imidazole éventuellement substitué par au moins un radical alkyle en C₁-C₁₄ ;
• un radical aromatique en C₆ relié aux groupements CR₃R₄ par des liaisons en position 1,3 l'une par rapport à l'autre, éventuellement substitué par un ou plusieurs atomes de fluor et/ou méthyle ; lesdites liaisons comprenant un radical alkyle, identique ou non, linéaire ou ramifié en C₁-C₄ et présentant un groupement comprenant un ou plusieurs hétéroatomes directement lié ou non au radical aromatique ; un radical aromatique en C₆ relié aux groupements CR₃R₄ par des liaisons en position 1,2 l'une par rapport à l'autre, lesdites liaisons comprenant un radical alkyle, identique ou non, linéaire ou ramifié en C₁-C₄ et présentant un groupement comprenant un atome d'oxygène directement lié au radical aromatique ; un radical aromatique en C₆ relié aux groupements CR₃R₄ par des liaisons en position 1,2 l'une par rapport à l'autre, substitué par un ou plusieurs groupements de type -C(=O)O- ; un radical aromatique en C₆ relié aux groupements CR₃R₄ par des liaisons en position 1,4 l'une par rapport à l'autre, lesdites liaisons comprenant un radical alkyle, identique ou non, linéaire ou ramifié en C₁-C₄ et présentant un groupement amide directement lié au radical aromatique ; un radical diphényle relié aux groupements CR₃R₄ par des liaisons en position 4,4' l'une par rapport à l'autre, les deux noyaux aromatiques étant éventuellement liés au moyen d'un radical alkyle, linéaire ou ramifié comprenant 1 à 4 atomes de carbone, de préférence 1 ou 2 atomes de carbone ;
• le groupement X pouvant porter une ou plusieurs charges cationiques.

33. Composé selon l'une quelconque des revendications 26 à 31, **caractérisé en ce que** X représente :
• un radical alkyle ramifié comprenant 4 à 13 atomes de carbone ; un radical alkyle linéaire ou ramifié comprenant 1 à 13 atomes de carbone, substitué par au moins un atome de chlore et/ou au moins un radical hydroxyle et/ou au moins un radical acétoxy et/ou au moins un radical amino et/ou au moins un radical ammonium; un radical alkyle linéaire ou ramifié comprenant 2 à 12 atomes de carbone interrompu par un ou plusieurs atomes d'oxygène, par un
ou plusieurs atomes d'azote porteurs d'un ou de deux radicaux, identiques ou non, choisis indépendamment les uns des autres, parmi les atomes d'hydrogène, les radicaux alkyle, linéaires ou ramifiés en C₁-C₄ éventuellement porteurs d'un groupement hydroxyle ; un radical alcène, linéaire ou ramifié, comprenant 2 à 12 atomes de carbone, et comprenant une liaison carbone-carbone insaturée ;
• un radical imidazole éventuellement substitué par au moins un radical alkyle en C₁-C₁₄ ;
• un radical aromatique en C₆ relié aux groupements CR₃R₄ par des liaisons en position 1,3 l'une par rapport à l'autre, éventuellement substitué par un ou plusieurs atomes de fluor et/ou méthyle ; lesdites liaisons comprenant un radical alkyle, identique ou non, linéaire ou ramifié en C₁-C₄ et présentant un groupement comprenant un ou plusieurs hétéroatomes directement lié ou non au radical aromatique ; un radical aromatique en C₆ relié aux groupements CR₃R₄ par des liaisons en position 1,4 l'une par rapport à l'autre, lesdites liaisons comprenant un radical alkyle, identique ou non, linéaire ou ramifié en C₁-C₄ et présentant un groupement amide directement lié au radical aromatique ;
• le groupement X pouvant porter une ou plusieurs charges cationiques.

34. Composé selon l'une quelconque des revendications 26 à 31, **caractérisé en ce que** :
les radicaux R₁ et R₂, identiques ou non, représentent un radical méthyle, un radical éthyle.
les radicaux R₅ et R₆ représentent un atome d'hydrogène
X représente :
• un radical alkyle ramifié comprenant 4 à 13 atomes de carbone ; un radical alkyle linéaire ou ramifié comprenant 1 à 13 atomes de carbone, substitué par au moins un atome de chlore et/ou au moins un radical hydroxyle et/ou au moins un radical acétoxy et/ou au moins un radical amino et/ou au moins un radical ammonium; un radical alkyle linéaire ou ramifié comprenant 2 à 12 atomes de carbone interrompu par un ou plusieurs atomes d'oxygène, par un ou plusieurs atomes d'azote porteurs d'un ou de deux radicaux, identiques ou non, choisis indépendamment les uns des autres, parmi les atomes d'hydrogène, les radicaux alkyle, linéaires ou ramifiés en C₁-C₄ éventuellement porteurs d'un groupement hydroxyle ; un radical alcène, linéaire ou ramifié, comprenant 2 à 12 atomes de carbone, et comprenant une liaison carbone-carbone insaturée ;
• un radical imidazole éventuellement substitué par au moins un radical alkyle en C₁-C₁₄ ;
• un radical aromatique en C₆ relié aux groupements CR₃R₄ par des liaisons en position 1,3 l'une par rapport à l'autre, éventuellement substitué par un ou plusieurs atomes de fluor et/ou méthyle ; lesdites liaisons comprenant un radical alkyle, identique ou non, linéaire ou ramifié en C₁-C₄ et présentant un groupement comprenant un ou plusieurs hétéroatomes directement lié ou non au radical aromatique ; un radical aromatique en C₆ relié aux groupements CR₃R₄ par des liaisons en position 1,4 l'une par rapport à l'autre, lesdites liaisons comprenant un radical alkyle, identique ou non, linéaire ou ramifié en C₁-C₄ et présentant un groupement amide directement lié au radical aromatique ;
• le groupement X pouvant porter une ou plusieurs charges cationiques.

35. Composition comprenant dans un milieu cosmétiquement acceptable au moins un composé fluorescent selon l'une des revendications 26 à 35.

36. Composition selon la revendication précédente, **caractérisée en ce que** la quantité en composé fluorescent est comprise entre 0,01 et 20% en poids, plus particulièrement entre 0,05 et 10% en poids, de préférence entre 0,1 et 5% en poids par rapport au poids total de la composition.

37. Composition selon l'une quelconque des revendications 35 ou 36, **caractérisée en ce que** la composition comprend au moins un colorant direct non fluorescent additionnel.

38. Composition selon la revendication précédente, **caractérisée en ce que** le ou les colorants directs additionnels sont choisis parmi les colorants benzéniques nitrés, les colorants azoïques, azométhiniques, méthiniques, anthraquinoniques, naphtoquinoniques, benzoquinoniques, phénotiaziniques, indigoïdes, xanthéniques, phénanthridiniques, phtalocyanines, ceux dérivés du triarylméthane seuls ou en mélanges.

39. Composition selon l'une quelconque des revendications 35 à 38, **caractérisée en ce que** la teneur en colorant direct additionnel est comprise entre 0,0005 et 12 % en poids, de préférence entre 0,005 à 6 % en poids, par rapport au poids total de la composition.

40. Composition selon l'une quelconque des revendications 35 à 39, **caractérisée en ce que** la composition comprend au moins un tensioactif non ionique, anionique, cationique, amphotère ou zwittérionique, ou leurs mélanges.

41. Composition selon la revendication précédente, **caractérisée en ce que** la teneur en tensioactif varie entre 0,01 et 40% en poids, de préférence entre 0,1 et 30% en poids, par rapport au poids total de la composition.

42. Composition selon l'une quelconque des revendications 35 à 41, **caractérisée en ce que** le milieu cosmétiquement acceptable comprend de l'eau et éventuellement un solvant organique.

43. Composition selon l'une quelconque des revendications 35 à 42, **caractérisée en ce qu'**elle se trouve sous la forme d'un shampooing colorant.

44. Composition selon l'une quelconque des revendications 35 à 43, **caractérisée en ce qu'**elle se trouve sous forme d'un mascara pour les cils ou d'un mascara capillaire.

45. Composition selon l'une quelconque des revendications 35 à 44, **caractérisée en ce que** la composition comprend au moins une base d'oxydation choisie parmi les paraphénylènediamines, les bis-phénylalkylènediamines, les para-aminophénols, les ortho-aminophénols et les bases hétérocycliques ou leurs sels d'addition avec un acide ou avec un agent alcalin.

46. Composition selon l'une quelconque des revendications 35 à 42 et 45, **caractérisée en ce que** la teneur en base d'oxydation varie entre 0,0005 et 12 % en poids, de préférence entre 0,005 et 6 % en poids, par rapport au poids total de la composition.

47. Composition selon l'une quelconque des revendications 35 à 42 et 45 ou 46, **caractérisée en ce qu'**elle comprend au moins un coupleur choisi parmi les métaphénylène-diamines, les méta-aminophénols, les métadiphénols et les coupleurs hétérocycliques ou leurs sels d'addition avec un acide ou avec un agent alcalin.

48. Composition selon l'une quelconque des revendications 35 à 42 et 45 à 47, **caractérisée en ce qu'**elle comprend une teneur en coupleur variant entre 0,0001 et 10 % en poids, de préférence entre 0,005 à 5 % en poids, par rapport au poids total de la composition.

49. Composition selon l'une quelconque des revendications 35 à 42 et 45 à 48, **caractérisée en ce que** la composition comprend au moins un agent oxydant.

50. Composition selon la revendication précédente, **caractérisée en ce que** l'agent oxydant est choisi parmi le peroxyde d'hydrogène, le peroxyde d'urée, les bromates de métaux alcalins, les persels tels que les perborates et persulfates, et les enzymes telles que les peroxydases et les oxydo-réductases à deux ou quatre électrons.

51. Dispositif à plusieurs compartiments, comprenant au moins un compartiment renfermant une composition selon l'une quelconque des revendications 35 à 42 et 45 à 47, comprenant au moins un composé fluorescent particulier et éventuellement au moins un colorant direct additionnel et/ou au moins une base d'oxydation et/ou au moins un coupleur, dans un milieu cosmétiquement acceptable, et au moins un autre compartiment renfermant une composition comprenant au moins un agent oxydant.

## Claims

1. Process for the dyeing, with a lightening effect, of human keratin materials, in which a composition is applied to the said materials, comprising, in a cosmetically acceptable medium, at least one fluorescent compound having the following formula: 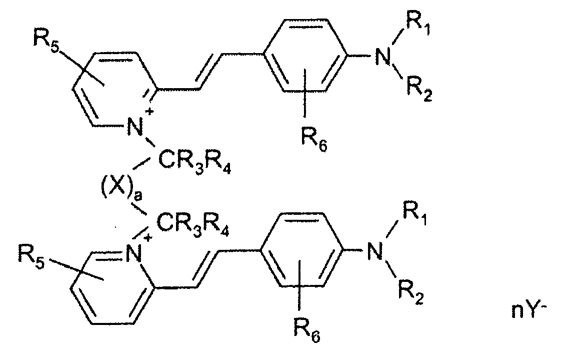 in which:
R₁ and R₂, which may be identical or different, represent:
• a hydrogen atom;
• a linear or branched alkyl radical containing 1 to 10 carbon atoms, optionally interrupted and/or substituted with at least one heteroatom and/or group comprising at least one heteroatom and/or substituted with at least one halogen atom;
• an aryl or arylalkyl radical, the aryl group containing 6 carbon atoms and the alkyl radical containing 1 to 4 carbon atoms; the aryl radical optionally being substituted with one or more linear or branched alkyl radicals comprising 1 to 4 carbon atoms optionally interrupted and/or substituted with at least one heteroatom and/or group comprising at least one heteroatom and/or substituted with at least one halogen atom;
• R₁ and R₂ may optionally be linked so as to form a heterocycle with the nitrogen atom and may comprise one or more other heteroatoms, the heterocycle optionally being substituted with at least one linear or branched alkyl radical preferably containing from 1 to 4 carbon atoms and optionally being interrupted and/or substituted with at least one heteroatom and/or group comprising at least one heteroatom and/or substituted with at least one halogen atom;
• R₁ or R₂ may optionally be engaged in a heterocycle comprising the nitrogen atom and one of the carbon atoms of the phenyl group bearing the said nitrogen atom;
R₃ and R₄, which may be identical or different, represent a hydrogen atom or an alkyl radical containing 1 to 4 carbon atoms;
R₅, which may be identical or different, represent a hydrogen atom, a halogen atom or a linear or branched alkyl radical containing 1 to 4 carbon atoms, optionally interrupted with at least one heteroatom;
R₆, which may be identical or different, represent a hydrogen atom; a halogen atom; a linear or branched alkyl radical containing 1 to 4 carbon atoms, optionally substituted and/or interrupted with at least one heteroatom and/or group bearing at least one heteroatom and/or substituted with at least one halogen atom;
X represents:
• a linear or branched alkyl radical containing 1 to 14 carbon atoms or an alkenyl radical containing 2 to 14 carbon atoms, optionally interrupted and/or substituted with at least one heteroatom and/or group containing at least one heteroatom and/or substituted with at least one halogen atom;
• a 5- or 6-membered heterocyclic radical optionally substituted with at least one linear or branched alkyl radical containing 1 to 14 carbon atoms, optionally substituted with at least one heteroatom; with at least one linear or branched aminoalkyl radical containing 1 to 4 carbon atoms, optionally substituted with at least one heteroatom; with at least one halogen atom;
• a fused or non-fused aromatic or diaromatic radical, optionally separated with an alkyl radical containing 1 to 4 carbon atoms, the aryl radical(s) optionally being substituted with at least one halogen atom or with at least one alkyl radical containing 1 to 10 carbon atoms optionally substituted and/or interrupted with at least one heteroatom and/or group bearing at least one heteroatom;
• a dicarbonyl radical;
• the group X possibly bearing one or more cationic charges;
a being equal to 0 or 1;
Y⁻, which may be identical or different, representing an organic or mineral anion;
n being an integer at least equal to 2 and at most equal to the number of cationic charges present in the fluorescent compound.

2. Process according to the preceding claim, **characterized in that** the compound is such that the radicals R₁ and R₂, which may be identical or different, represent:
• a hydrogen atom;
• an alkyl radical containing 1 to 6 carbon atoms, optionally interrupted with an oxygen atom or optionally substituted with at least one hydroxyl, amino or ammonium radical or a chlorine or fluorine atom;
• a benzyl or phenyl radical, optionally substituted with an alkyl or alkoxy radical containing 1 to 4 carbon atoms and preferably 1 or 2 carbon atoms;
• with the nitrogen atom, a heterocyclic radical of the pyrrolo, pyrrolidino, imidazolino, imidazolo, imidazolium, pyrazolino, piperazino, morpholino, morpholo, pyrazolo or triazolo type, optionally substituted with at least one linear or branched alkyl radical containing 1 to 4 carbon atoms optionally interrupted and/or substituted with a nitrogen and/or oxygen atom and/or a group bearing a nitrogen and/or oxygen atom.

3. Process according to either of the preceding claims, **characterized in that** the radicals R₁ and R₂, which may be identical or different, and are preferably identical, represent an alkyl radical containing 1 to 4 carbon atoms.

4. Process according to any one of the preceding claims, **characterized in that** the radicals R₁ and R₂, which may be identical or different, and are preferably identical, represent a methyl radical or an ethyl radical.

5. Process according to any one of the preceding claims, **characterized in that** the radicals R₅ and R₆ represent a hydrogen atom.

6. Process according to any one of the preceding claims, **characterized in that** the compound is such that the group X represents:
• a linear or branched alkyl radical containing 1 to 14 carbon atoms or an alkenyl radical containing 2 to 14 carbon atoms, optionally substituted and/or interrupted with one or more oxygen and/or nitrogen atoms, and/or with one or more groups bearing at least one heteroatom, for instance hydroxyl, alkoxy, amino, ammonium, amido, carbonyl or carboxyl (-COO-, -O-CO-) groups, and/or with a fluorine atom;
• a dicarbonyl radical;
• a 5- or 6-membered heterocyclic radical, of the imidazolo, pyrazolo, triazino or pyridino type, optionally substituted with at least one linear or branched alkyl radical containing 1 to 14 carbon atoms, more particularly 1 to 10 carbon atoms and preferably from 1 to 4 carbon atoms; with at least one linear or branched aminoalkyl radical containing 1 to 10 carbon atoms and preferably from 1 to 4 carbon atoms, optionally substituted with a group comprising at least one heteroatom, for instance a hydroxyl radical, with at least one halogen atom;
• an aromatic radical containing 6 carbon atoms or a fused or non-fused diaromatic radical especially containing from 10 to 12 carbon atoms, optionally separated with an alkyl radical containing 1 to 4 carbon atoms, the aryl radical(s) being optionally substituted with at least one halogen atom and/or with at least one alkyl radical containing 1 to 10 carbon atoms optionally interrupted with at least one oxygen and/or nitrogen atom, and/or a group comprising at least one heteroatom, for instance a carbonyl, carboxyl, amido, amino or ammonium radical.

7. Process according to any one of the preceding claims, **characterized in that** the composition comprises an amount of fluorescent compound of between 0.01% and 20% by weight, more particularly between 0.05% and 10% by weight and preferably between 0.1% and 5% by weight relative to the total weight of the composition.

8. Process according to any one of the preceding claims, **characterized in that** the composition comprises at least one additional fluorescent compound.

9. Process according to the preceding claim, **characterized in that** the content of additional fluorescent compound is between 0.05% and 10% by weight relative to the weight of the composition, and preferably between 0.1% and 5% by weight relative to the weight of the composition.

10. Process according to any one of the preceding claims, **characterized in that** the composition comprises at least one additional non-fluorescent direct dye.

11. Process according to the preceding claim, **characterized in that** the additional direct dye(s) is (are) chosen from nitrobenzene dyes, azo, azomethine, methine, anthraquinone, naphthoquinone, benzoquinone, phenothiazine, indigoid, xanthene, phenanthridine, phthalocyanin and triarylmethane-based dyes, alone or as mixtures.

12. Process according to any one of the preceding claims, **characterized in that** the composition comprises a content of additional direct dye of between 0.0005% and 12% by weight and preferably between 0 . 005 % and 6% by weight relative to the total weight of the composition.

13. Process according to any one of the preceding claims, **characterized in that** the composition comprises at least one nonionic, anionic, cationic, amphoteric or zwitterionic surfactant, or mixtures thereof.

14. Process according to the preceding claim, **characterized in that** the composition comprises a surfactant content ranging between 0.01% and 40% by weight and preferably between 0.1% and 30% by weight relative to the total weight of the composition.

15. Process according to any one of the preceding claims, **characterized in that** the cosmetically acceptable medium comprises water and optionally an organic solvent.

16. Process according to any one of the preceding claims, **characterized in that** the composition is in the form of a colouring shampoo.

17. Process according to any one of Claims 1 to 15, **characterized in that** the composition is in the form of an eyelash mascara or a hair mascara.

18. Process according to any one of Claims 1 to 15, **characterized in that** the composition comprises at least one oxidation base chosen from para-phenylenediamines, bis(phenyl)alkylenediamines, para-aminophenols, ortho-aminophenols and heterocyclic bases, or the addition salts thereof with an acid or with an alkaline agent.

19. Process according to any one of Claims 1 to 15 and 18, **characterized in that** the composition comprises a content of oxidation base ranging between 0.0005% and 12% by weight and preferably between 0.005% and 6% by weight relative to the total weight of the composition.

20. Process according to any one of Claims 1 to 15, 18 and 19, **characterized in that** the composition comprises at least one coupler chosen from meta-phenylenediamines, meta-aminophenols, meta-diphenols and heterocyclic couplers, or the addition salts thereof with an acid or with an alkaline agent.

21. Process according to any one of Claims 1 to 15 and 18 to 20, **characterized in that** the composition comprises a coupler content ranging between 0.0001% and 10% by weight and preferably between 0.005% and 5% by weight relative to the total weight of the composition.

22. Process according to any one of Claims 1 to 15 and 18 to 21, **characterized in that** the composition comprises at least one oxidizing agent.

23. Process according to the preceding claim, **characterized in that** the oxidizing agent is chosen from hydrogen peroxide, urea peroxide, alkali metal bromates, persalts such as perborates and persulfates, and enzymes such as peroxidases and two-electron or four-electron oxidoreductases.

24. Process according to one of Claims 1 to 15 and 17, **characterized in that** the following steps are performed:
a) the composition is applied to human keratin materials,
b) the materials are dried or are left to dry.

25. Process according to any one of Claims 1 to 16 and 18 to 23, **characterized in that** the following steps are performed:
a) the composition is applied to human keratin materials, for a time that is sufficient to develop the desired coloration and lightening,
b) the materials thus treated are optionally rinsed,
c) the said materials are optionally washed and rinsed,
d) the materials are dried or are left to dry.

26. Fluorescent compound having the following formula: 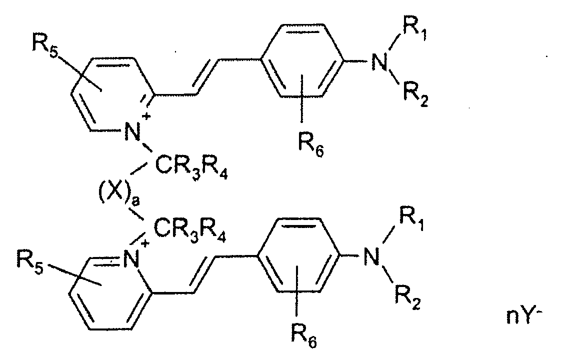 in which:
R₁ and R₂, which may be identical or different, represent:
• a hydrogen atom;
• a linear or branched alkyl radical containing 1 to 10 carbon atoms, optionally interrupted and/or substituted with at least one heteroatom and/or group comprising at least one heteroatom and/or substituted with at least one halogen atom;
• an aryl or arylalkyl radical, the aryl group containing 6 carbon atoms and the alkyl radical containing 1 to 4 carbon atoms; the aryl radical optionally being substituted with one or more linear or branched alkyl radicals comprising 1 to 4 carbon atoms optionally interrupted and/or substituted with at least one heteroatom and/or group comprising at least one heteroatom and/or substituted with at least one halogen atom;
• R₁ and R₂ may optionally be linked so as to form a heterocycle with the nitrogen atom and may comprise one or more other heteroatoms, the heterocycle optionally being substituted with at least one linear or branched alkyl radical preferably containing from 1 to 4 carbon atoms and optionally being interrupted and/or substituted with at least one heteroatom and/or group comprising at least one heteroatom and/or substituted with a halogen atom;
• R₁ or R₂ may optionally be engaged in a heterocycle comprising the nitrogen atom and one of the carbon atoms of the phenyl group bearing the said nitrogen atom;
R₃ and R₄, which may be identical or different, represent a hydrogen atom or an alkyl radical containing 1 to 4 carbon atoms;
R₅, which may be identical or different, represent a hydrogen atom, a halogen atom or a linear or branched alkyl radical containing 1 to 4 carbon atoms, optionally interrupted with at least one heteroatom;
R₆, which may be identical or different, represent a hydrogen atom; a halogen atom; a linear or branched alkyl radical containing 1 to 4 carbon atoms, optionally substituted and/or interrupted with at least one heteroatom and/or group bearing at least one heteroatom and/or substituted with at least one halogen atom;
X represents:
• a linear or branched alkyl radical containing 1 to 14 carbon atoms or an alkenyl radical containing 2 to 14 carbon atoms, optionally interrupted and/or substituted with at least one heteroatom and/or group containing at least one heteroatom and/or substituted with at least one halogen atom;
• a 5- or 6-membered heterocyclic radical optionally substituted with at least one linear or branched alkyl radical containing 1 to 14 carbon atoms, optionally substituted with at least one heteroatom; with at least one linear or branched aminoalkyl radical containing 1 to 4 carbon atoms, optionally substituted with at least one heteroatom; with at least one halogen atom;
• a fused or non-fused aromatic or diaromatic radical, optionally separated with an alkyl radical containing 1 to 4 carbon atoms, the aryl radical (s) optionally being substituted with at least one halogen atom or with at least one alkyl radical containing 1 to 10 carbon atoms optionally substituted and/or interrupted with at least one heteroatom and/or group bearing at least one heteroatom;
• a dicarbonyl radical;
• the group X possibly bearing one or more cationic charges;
a being equal to 0 or 1;
Y⁻, which may be identical or different, representing an organic or mineral anion;
n being an integer at least equal to 2 and at most equal to the number of cationic charges present in the fluorescent compound;
with the exception of compounds for which:
• the group X represents an unsubstituted linear alkyl radical containing 1 or 4 carbon atoms with a equal to 1 or a equal to 0; and the radicals R₁ and R₂ simultaneously represent a methyl radical; R₅ and R₆ represent a hydrogen atom; R₃ and R₄, which are identical, representing a hydrogen atom;
• the group X represents a linear unsubstituted C₂ alkyl radical or a linear or branched unsubstituted C₃ alkyl radical; a is equal to 1; R₃ and R₄, which are identical, representing a hydrogen atom; R₅ represents a hydrogen atom; the radicals R₁ and R₂:
- which are either identical, represent a methyl radical, R₆ represents a hydrogen atom, or a methyl radical in an ortho position relative to the carbon atom of the benzene ring bearing the unsaturated carbon-carbon bond; or R₁ and R₂, which are identical, represent an ethyl radical, R₆ represents a hydrogen atom, or a methoxy radical in an ortho position relative to the carbon atom of the benzene ring bearing the unsaturated carbon-carbon bond;
- or are different, represent an ethyl radical and an ethyl radical substituted with a dimethylamino, trimethylammonium or benzyldimethylammonium group;
• the group X represents a phenyl radical linked to the groups CR₃R₄ via bonds in the 1,4 position relative to each other; R₃ and R₄, which are identical, represent a hydrogen atom; a is equal to 1; R₅ represents a hydrogen atom; the radicals R₁ and R₂:
- which are either identical, represent a methyl radical, R₆ represents a hydrogen atom, or a methyl radical in an ortho position relative to the carbon atom of the benzene ring bearing the unsaturated carbon-carbon bond; or R₁ and R₂, which are identical, represent an ethyl radical, R₆ represents a hydrogen atom, or a methoxy radical in an ortho position relative to the carbon atom of the benzene ring bearing the unsaturated carbon-carbon bond;
- or are different, represent an ethyl radical and an ethyl radical substituted with a dimethylamino, trimethylammonium or benzyldimethylammonium group;

27. Compound according to Claim 26, **characterized in that** the radicals R₁ and R₂, which may be identical or different, represent:
• a hydrogen atom;
• an alkyl radical containing 1 to 6 carbon atoms, optionally interrupted with an oxygen atom or optionally substituted with at least one hydroxyl, amino or ammonium radical or a chlorine or fluorine atom;
• a benzyl or phenyl radical, optionally substituted with an alkyl or alkoxy radical containing 1 to 4 carbon atoms and preferably 1 or 2 carbon atoms;
• with the nitrogen atom, a heterocyclic radical of the pyrrolo, pyrrolidino, imidazolino, imidazolo, imidazolium, pyrazolino, piperazino, morpholino, morpholo, pyrazolo or triazolo type, optionally substituted with at least one linear or branched alkyl radical containing 1 to 4 carbon atoms optionally interrupted and/or substituted with a nitrogen and/or oxygen atom and/or a group bearing a nitrogen and/or oxygen atom.

28. Compound according to either of Claims 26 and 27, **characterized in that** the radicals R₁ and R₂, which may be identical or different, represent an alkyl radical containing 1 to 4 carbon atoms.

29. Compound according to the preceding claim, **characterized in that** the radicals R₁ and R₂, which may be identical or different, represent a methyl radical or an ethyl radical.

30. Compound according to any one of Claims 26 to 29, **characterized in that** the radicals R₅ and R₆ represent a hydrogen atom.

31. Compound according to any one of Claims 26 to 30, **characterized in that** X represents:
• a branched alkyl radical containing 4 to 14 carbon atoms; a linear or branched alkyl radical containing 1 to 14 carbon atoms, substituted with one or more halogen atoms and/or heteroatoms and/or groups bearing one or more heteroatoms; a linear or branched alkyl radical containing 1 to 14 carbon atoms interrupted with one or more heteroatoms and/or groups bearing one or more heteroatoms; a linear or branched alkene radical containing 2 to 14 carbon atoms, optionally interrupted and/or substituted with one or more heteroatoms or groups bearing one or more heteroatoms;
• a dicarbonyl radical;
• a 5- or 6-membered heterocyclic radical, optionally substituted with at least one alkyl radical containing 1 to 14 carbon atoms, optionally substituted with at least one heteroatom; with at least one linear or branched aminoalkyl radical containing 1 to 4 carbon atoms, optionally substituted with at least one heteroatom; with at least one halogen atom;
• a C₆ aromatic radical linked to the groups CR₃R₄ via bonds in the 1,3 position relative to each other, optionally substituted with one or more halogen atoms and/or C₁-C₄ alkyl radicals; the said bonds comprising an identical or different, linear or branched C₁-C₄ alkyl radical and containing a group comprising one or more heteroatoms which may or may not be directly linked to the aromatic radical; a C₆ aromatic radical linked to the groups CR₃R₄ via bonds in the 1,2 position relative to each other, the said bonds comprising an identical or different, linear or branched C₁-C₄ alkyl radical and containing a group comprising one or more heteroatoms which may or may not be directly linked to the aromatic radical; a C₆ aromatic radical linked to the groups CR₃R₄ via bonds in the 1,2 position relative to each other, substituted with one or more groups comprising one or more heteroatoms; a C₆ aromatic radical linked to the groups CR₃R₄ via bonds in the 1,4 position relative to each other, the said bonds comprising an identical or different, linear or branched C₁-C₄ alkyl radical and containing a group comprising one or more heteroatoms which may or may not be directly linked to the aromatic radical; a diphenyl radical linked to the groups CR₃R₄ via bonds in the 4,4' position relative to each other, the two aromatic nuclei being optionally linked by means of a linear or branched C₁-C₄ alkyl radical;
• the group X possibly bearing one or more cationic charges.

32. Compound according to any one of Claims 26 to 31, **characterized in that** X represents:
• a branched alkyl radical containing 4 to 13 carbon atoms; a linear, or branched alkyl radical containing 1 to 13 carbon atoms, substituted with at least one chlorine atom and/or at least one hydroxyl radical and/or at least one acetoxy radical and/or at least one amino radical and/or at least one ammonium radical; a linear or branched alkyl radical containing 2 to 12 carbon atoms interrupted with one or more oxygen atoms, with one or more nitrogen atoms bearing one or two radicals, which may be identical or different, chosen, independently of each other, from hydrogen atoms and linear or branched C₁-C₄ alkyl radicals optionally bearing a hydroxyl group; a linear or branched alkene radical containing 2 to 12 carbon atoms and comprising an unsaturated carbon-carbon bond;
• a pyridine radical linked to the groups CR₃R₄ via bonds in positions 2 and 6 relative to each other;
• an imidazole radical optionally substituted with at least one C₁-C₁₄ alkyl radical;
• a C₆ aromatic radical linked to the groups CR₃R₄ via bonds in the 1,3 position relative to each other, optionally substituted with one or more fluorine atoms and/or methyl; the said bonds comprising an identical or different, linear or branched C₁-C₄ alkyl radical and containing a group comprising one or more heteroatoms which may or may not be directly linked to the aromatic radical; a C₆ aromatic radical linked to the groups CR₃R₄ via bonds in the 1,2 position relative to each other, the said bonds comprising an identical or different, linear or branched C₁-C₄ alkyl radical and containing a group comprising an oxygen atom directly linked to the aromatic radical; a C₆ aromatic radical linked to the groups CR₃R₄ via bonds in the 1,2 position relative to each other, substituted with one or more groups of the type -C (=O) O-; a C₆ aromatic radical linked to the groups CR₃R₄ via bonds in the 1,4 position relative to each other, the said bonds comprising an identical or different, linear or branched C₁-C₄ alkyl radical and containing an amide group directly linked to the aromatic radical; a diphenyl radical linked to the groups CR₃R₄ via bonds in the 4,4' position relative to each other, the two aromatic nuclei being optionally linked by means of a linear or branched alkyl radical containing 1 to 4 carbon atoms and preferably 1 or 2 carbon atoms;
• the group X possibly bearing one or more cationic charges.

33. Compound according to any one of Claims 26 to 31, **characterized in that** X represents:
• a branched alkyl radical containing 4 to 13 carbon atoms; a linear or branched alkyl radical containing 1 to 13 carbon atoms, substituted with at least one chlorine atom and/or at least one hydroxyl radical and/or at least one acetoxy radical and/or at least one amino radical and/or at least one ammonium radical; a linear or branched alkyl radical containing 2 to 12 carbon atoms interrupted with one or more oxygen atoms, with one or more nitrogen atoms bearing one or two radicals, which may be identical or different, chosen, independently of each other, from hydrogen atoms and linear or branched C₁-C₄ alkyl radicals optionally bearing a hydroxyl group; a linear or branched alkene radical containing 2 to 12 carbon atoms and comprising an unsaturated carbon-carbon bond;
• an imidazole radical optionally substituted with at least one C₁-C₁₄ alkyl radical;
• a C₆ aromatic radical linked to the groups CR₃R₄ via bonds in the 1,3 position relative to each other, optionally substituted with one or more fluorine atoms and/or methyl; the said bonds comprising an identical or different, linear or branched C₁-C₄ alkyl radical and containing a group comprising one or more heteroatoms which may or may not be directly linked to the aromatic radical; a C₆ aromatic radical linked to the groups CR₃R₄ via bonds in the 1,4 position relative to each other, the said bonds comprising an identical or different, linear or branched C₁-C₄ alkyl radical and containing an amide group directly linked to the aromatic radical;
• the group X possibly bearing one or more cationic charges.

34. Compound according to any one of Claims 26 to 31, **characterized in that**:
the radicals R₁ and R₂, which may be identical or different, represent a methyl radical or an ethyl radical,
the radicals R₅ and R₆ represent a hydrogen atom,
X represents:
• a branched alkyl radical containing 4 to 13 carbon atoms; a linear or branched alkyl radical containing 1 to 13 carbon atoms, substituted with at least one chlorine atom and/or at least one hydroxyl radical and/or at least one acetoxy radical and/or at least one amino radical and/or at least one ammonium radical; a linear or branched alkyl radical containing 2 to 12 carbon atoms interrupted with one or more oxygen atoms, with one or more nitrogen atoms bearing one or two radicals, which may be identical or different, chosen, independently of each other, from hydrogen atoms and linear or branched C₁-C₄ alkyl radicals optionally bearing a hydroxyl group; a linear or branched alkene radical containing 2 to 12 carbon atoms and comprising an unsaturated carbon-carbon bond;
• an imidazole radical optionally substituted with at least one C₁-C₁₄ alkyl radical;
• a C₆ aromatic radical linked to the groups CR₃R₄ via bonds in the 1,3 position relative to each other, optionally substituted with one or more fluorine atoms and/or methyl; the said bonds comprising an identical or different, linear or branched C₁-C₄ alkyl radical and containing a group comprising one or more heteroatoms which may or may not be directly linked to the aromatic radical; a C₆ aromatic radical linked to the groups CR₃R₄ via bonds in the 1,4 position relative to each other, the said bonds comprising an identical or different, linear or branched C₁-C₄ alkyl radical and containing an amide group directly linked to the aromatic radical;
• the group X possibly bearing one or more cationic charges.

35. Composition comprising, in a cosmetically acceptable medium, at least one fluorescent compound according to one of Claims 26 to 35.

36. Composition according to the preceding claim, **characterized in that** the amount of fluorescent compound is between 0.01% and 20% by weight, more particularly between 0.05% and 10% by weight and preferably between 0.1% and 5% by weight relative to the total weight of the composition.

37. Composition according to either of Claims 35 and 36, **characterized in that** the composition comprises at least one additional non-fluorescent direct dye.

38. Composition according to the preceding claim, **characterized in that** the additional direct dye(s) is (are) chosen from nitrobenzene dyes, azo, azomethine, methine, anthraquinone, naphthoquinone, benzoquinone, phenothiazine, indigoid, xanthene, phenanthridine, phthalocyanin and triarylmethane-based dyes, alone or as mixtures.

39. Composition according to any one of Claims 35 to 38, **characterized in that** the content of additional direct dye is between 0.0005% and 12% by weight and preferably between 0.005% and 6% by weight relative to the total weight of the composition.

40. Composition according to any one of Claims 35 to 39, **characterized in that** the composition comprises at least one nonionic, anionic, cationic, amphoteric or zwitterionic surfactant, or mixtures thereof.

41. Composition according to the preceding claim, **characterized in that** the surfactant content ranges between 0.01% and 40% by weight and preferably between 0.1% and 30% by weight relative to the total weight of the composition.

42. Composition according to any one of Claims 35 to 41, **characterized in that** the cosmetically acceptable medium comprises water and optionally an organic solvent.

43. Composition according to any one of Claims 35 to 42, **characterized in that** it is in the form of a colouring shampoo.

44. Composition according to any one of Claims 35 to 43, **characterized in that** it is in the form of an eyelash mascara or a hair mascara.

45. Composition according to any one of Claims 35 to 44, **characterized in that** the composition comprises at least one oxidation base chosen from para-phenylenediamines, bis(phenyl)alkylenediamines, para-aminophenols, ortho-aminophenols and heterocyclic bases, or the addition salts thereof with an acid or with an alkaline agent.

46. Composition according to any one of Claims 35 to 42 and 45, **characterized in that** the content of oxidation base ranges between 0.0005% and 12% by weight and preferably between 0.005% and 6% by weight relative to the total weight of the composition.

47. Composition according to any one of Claims 35 to 42, 45 and 46, **characterized in that** it comprises at least one coupler chosen from meta-phenylenediamines, meta-aminophenols, meta-diphenols and heterocyclic couplers, or the addition salts thereof with an acid or with an alkaline agent.

48. Composition according to any one of Claims 35 to 42 and 45 to 47, **characterized in that** it comprises a coupler content ranging between 0.0001% and 10% by weight and preferably between 0.005% and 5% by weight relative to the total weight of the composition.

49. Composition according to any one of Claims 35 to 42 and 45 to 48, **characterized in that** the composition comprises at least one oxidizing agent.

50. Composition according to the preceding claim, **characterized in that** the oxidizing agent is chosen from hydrogen peroxide, urea peroxide, alkali metal bromates, persalts such as perborates and persulfates, and enzymes such as peroxidases and two-electron or four-electron oxidoreductases.

51. Multi-compartment device comprising at least one compartment containing a composition according to any one of Claims 35 to 42 and 45 to 47, comprising at least one particular fluorescent compound and optionally at least one additional direct dye and/or at least one oxidation base and/or at least one coupler, in a cosmetically acceptable medium, and at least one other compartment containing a composition comprising at least one oxidizing agent.

## Patentansprüche

1. Verfahren zum Färben von menschlichen Keratinsubstanzen mit einer aufhellenden Wirkung, wobei auf die Keratinsubstanzen eine Zusammensetzung aufgetragen wird, die in einem kosmetisch akzeptablen Medium mindestens eine fluoreszierende Verbindung der folgenden Formel enthält: 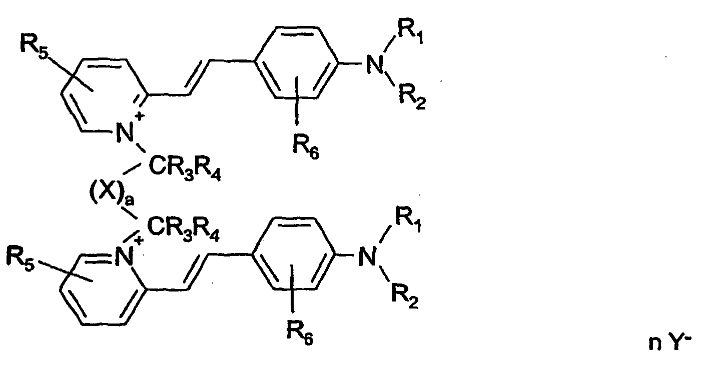 worin bedeuten:
die Gruppen R₁ und R₂, die gleich oder verschieden sind:
· ein Wasserstoffatom;
· eine geradkettige oder verzweigte Alkylgruppe mit 1 bis 10 Kohlenstoffatomen, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
· eine Aryl- oder Arylalkylgruppe, wobei die Arylgruppe 6 Kohlenstoffatome und die Alkylgruppe 1 bis 4 Kohlenstoffatome besitzt; wobei die Arylgruppe gegebenenfalls mit einer oder mehreren, geradkettigen oder verzweigten Alkylgruppen mit 1 bis 4 Kohlenstoffatomen substituiert ist, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert sind und/oder mit mindestens einem Halogenatom substituiert sind;
· die Gruppen R₁ und R₂ können gegebenenfalls so verbunden sein, dass sie mit dem Stickstoffatom einen Heterocyclus bilden, wobei ein oder mehrere weitere Heteroatome enthalten sein können, wobei der Heterocyclus gegebenenfalls mit mindestens einer geradkettigen oder verzweigten Alkylgruppe substituiert ist, die vorzugsweise 1 bis 4 Kohlenstoffatome enthält und gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
· die Gruppe R₁ oder R₂ kann gegebenenfalls in einen Heterocyclus eingebunden sein, der das Stickstoffatom und ein Kohlenstoffatom des Phenylrings, der das Stickstoffatom trägt, enthält.
die Gruppen R₃ und R₄, die identisch oder verschieden sind, ein Wasserstoffatom oder eine Alkylgruppe mit 1 bis 4 Kohlenstoffatomen;
die Gruppen R₅, die gleich oder verschieden sind, ein Wasserstoffatom; ein Halogenatom; eine geradkettige oder verzweigte Alkylgruppe mit 1 bis 4 Kohlenstoffatomen, die gegebenenfalls durch mindestens ein Heteroatom unterbrochen ist;
die Gruppen R₆, die gleich oder verschieden sind, ein Wasserstoffatom; ein Halogenatom; eine geradkettige oder verzweigte Alkylgruppe mit 1 bis 4 Kohlenstoffatomen, die gegebenenfalls mit mindestens einem Heteroatom und/oder einer Gruppe, die mindestens ein Heteroatom enthält, substituiert und/oder durch mindestens ein Heteroatom und/oder eine Gruppe, die mindestens ein Heteroatom enthält, unterbrochen ist und/oder mit mindestens einem Halogenatom substituiert ist;
X bedeutet:
· eine geradkettige oder verzweigte Alkylgruppe mit 1 bis 14 Kohlenstoffatomen oder eine Alkenylgruppe mit 2 bis 14 Kohlenstoffatomen, die gegebenenfalls durch mindestens ein Heteroatom und/ oder eine Gruppe, die mindestens ein Heteroatom enthält, unterbrochen und/oder mit mindestens einem Heteroatom und/oder einer Gruppe, die mindestens ein Heteroatom enthält, substituiert und/oder mit mindestens einem Halogenatom substituiert sind;
· eine 5- oder 6-gliedrige heterocyclische Gruppe, die gegebenenfalls mit mindestens einer geradkettigen oder verzweigten Alkylgruppe mit 1 bis 14 Kohlenstoffatomen, die gegebenenfalls mit mindestens einem Heteroatom substituiert ist; mit mindestens einer geradkettigen oder verzweigten Aminoalkylgruppe mit 1 bis 4 Kohlenstoffatomen, die gegebenenfalls mit mindestens einem Heteroatom substituiert ist; mit mindestens einem Halogenatom substituiert ist;
· eine aromatische Gruppe oder eine kondensierte oder nicht kondensierte, diaromatische Gruppe, die gegebenenfalls über eine Alkylgruppe mit 1 bis 4 Kohlenstoffatomen getrennt ist, wobei die Arylgruppe(n) gegebenenfalls mit mindestens einem Halogenatom oder mit mindestens einer Alkylgruppe mit 1 bis 10 Kohlenstoffatomen substituiert sind, die gegebenenfalls mit mindestens einem Heteroatom und/oder einer Gruppe, die mindestens ein Heteroatom enthält, substituiert und/oder durch mindestens ein Heteroatom und/oder eine Gruppe, die mindestens ein Heteroatom enthält, unterbrochen ist;
· eine Dicarbonylgruppe;
· wobei die Gruppe X eine oder mehrere kationische Ladungen umfassen kann;
a 0 oder 1;
die Gruppen Y⁻, die gleich oder verschieden sind, ein organisches oder anorganisches Anion;
wobei n eine ganze Zahl ist, die mindestens 2 und höchstens die Anzahl der in der fluoreszierenden Verbindung vorhandenen kationischen Ladungen bedeutet.

2. Verfahren nach dem vorhergehenden Anspruch, **dadurch gekennzeichnet, dass** die Verbindung so vorliegt, dass die Gruppen R₁ und R₂, die gleich oder verschieden sind, bedeuten:
· ein Wasserstoffatom;
· eine Alkylgruppe mit 1 bis 6 Kohlenstoffatomen, die gegebenenfalls durch ein Sauerstoffatom unterbrochen oder gegebenenfalls mit mindestens einer Hydroxygruppe, einer Aminogruppe, einer Ammoniumgruppe, einem Chloratom oder einem Fluoratom substituiert ist;
· eine Benzylgruppe, eine Phenylgruppe, die gegebenenfalls mit einer Alkyl- oder Alkoxygruppe mit 1 bis 4 Kohlenstoffatomen und vorzugsweise 1 oder 2 Kohlenstoffatomen substituiert sind;
· zusammen mit dem Stickstoffatom eine heterocyclische Gruppe vom Typ Pyrrolo, Pyrrolidino, Imidazolino, Imidazolo, Imidazolium, Pyrazolino, Piperazino, Morpholino, Morpholo, Pyrazolo, Triazolo, die gegebenenfalls mit mindestens einer geradkettigen oder verzweigten Alkylgruppe mit 1 bis 4 Kohlenstoffatomen substituiert ist, die gegebenenfalls mit einem Stickstoffatom und/oder Sauerstoffatom und/oder einer Gruppe, die ein Stickstoff- und/oder Sauerstoffatom enthält, substituiert und/oder durch ein Stickstoffatom und/oder durch ein Sauerstoffatom und/oder eine Gruppe, die ein Stickstoff- und/oder Sauerstoffatom enthält, unterbrochen ist.

3. Verfahren nach einem der vorhergehenden Ansprüche, **dadurch gekennzeichnet, dass** die Gruppen R₁ und R₂, die gleich oder verschieden und vorzugsweise gleich sind, eine Alkylgruppe mit 1 bis 4 Kohlenstoffatomen bedeuten.

4. Verfahren nach einem der vorhergehenden Ansprüche, **dadurch gekennzeichnet, dass** die Gruppen R₁ und R₂, die gleich oder verschieden und vorzugsweise gleich sind, eine Methylgruppe oder eine Ethylgruppe bedeuten.

5. Verfahren nach einem der vorhergehenden Ansprüche, **dadurch gekennzeichnet, dass** die Gruppen R₅ und R₆ ein Wasserstoffatom bedeuten.

6. Verfahren nach einem der vorhergehenden Ansprüche, **dadurch gekennzeichnet, dass** die Verbindung so vorliegt, dass die Gruppe X bedeutet:
· eine geradkettige oder verzweigte Alkylgruppe mit 1 bis 14 Kohlenstoffatomen oder eine Alkenylgruppe mit 2 bis 14 Kohlenstoffatomen, die durch ein oder mehrere Sauerstoffatome und/oder Stickstoffatome und/oder eine oder mehrere Gruppen, die mindestens ein Heteroatom enthalten, wie die Gruppen Hydroxy, Alkoxy, Amino, Ammonium, Amido, Carbonyl, Carboxy (-COO-, -O-CO-), substituiert und/oder durch solche Atome und/oder Gruppen unterbrochen sind und/oder mit einem Fluoratom substituiert sind;
· eine Dicarbonylgruppe;
· eine 5- oder 6-gliedrige heterocyclische Gruppe vom Typ Imidazolo, Pyrazolo, Triazino, Pyridino, die gegebenenfalls mit mindestens einer geradkettigen oder verzeigten Alkylgruppe mit 1 bis 14 Kohlenstoffatomen, insbesondere 1 bis 10 Kohlenstoffatomen und vorzugsweise 1 bis 4 Kohlenstoffatomen; mit mindestens einer geradkettigen oder verzweigten Aminoalkylgruppe mit 1 bis 10 Kohlenstoffatomen und vorzugsweise 1 bis 4 Kohlenstoffatomen, die gegebenenfalls mit einer Gruppe substituiert ist, die mindestens ein Heteroatom enthält, wie Hydroxy; mit mindestens einem Halogenatom substituiert ist;
· eine aromatische Gruppe mit 6 Kohlenstoffatomen oder eine kondensierte oder nicht kondensierte diaromatische Gruppe mit insbesondere 10 bis 12 Kohlenstoffatomen, die gegebenenfalls über eine Alkylgruppe mit 1 bis 4 Kohlenstoffatomen getrennt wird, wobei die Arylgruppe(n) gegebenenfalls mit mindestens einem Halogenatom und/oder mit mindestens einer Alkylgruppe mit 1 bis 10 Kohlenstoffatomen, die gegebenenfalls durch mindestens ein Sauerstoffatom und/oder ein Stickstoffatom unterbrochen ist, und/oder einer Gruppe, die mindestens ein Heteroatom enthält, wie Carbonyl, Carboxy, Amido, Amino, Ammonium, substituiert sind.

7. Verfahren nach einem der vorhergehenden Ansprüche, **dadurch gekennzeichnet, dass** die Zusammensetzung einen Mengenanteil der fluoreszierenden Verbindung im Bereich von 0,01 bis 20 Gew.-%, insbesondere 0,05 bis 10 Gew.-% und vorzugsweise 0,1 bis 5 Gew.-%, bezogen auf das Gesamtgewicht der Zusammensetzung, enthält.

8. Verfahren nach einem der vorhergehenden Ansprüche, **dadurch gekennzeichnet, dass** die Zusammensetzung ferner mindestens eine ergänzende fluoreszierende Verbindung enthält.

9. Verfahren nach dem vorhergehenden Anspruch, **dadurch gekennzeichnet, dass** der Mengenanteil der ergänzenden fluoreszierenden Verbindung im Bereich von 0,05 bis 10 Gew.-%, bezogen auf das Gewicht der Zusammensetzung, und vorzugsweise 0,1 bis 5 Gew.-%, bezogen auf das Gewicht der Zusammensetzung, liegt.

10. Verfahren nach einem der vorhergehenden Ansprüche, **dadurch gekennzeichnet, dass** die Zusammensetzung ferner mindestens einen nicht fluoreszierenden zusätzlichen Direktfarbstoff enthält.

11. Verfahren nach dem vorhergehenden Anspruch, **dadurch gekennzeichnet, dass** der oder die ergänzenden Direktfarbstoffe unter den nitrierten Benzolfarbstoffen, Azofarbstoffen, Azomethin-Farbstoffen, Methin-Farbstoffen, Anthrachinon-Farbstoffen, Naphthochinon-Farbstoffen, Benzochinon-Farbstoffen, Phenothiazin-Farbstoffen, Indigoiden, Xanthen-Farbstoffen, Phenanthridin-Farbstoffen, Phthalocyaninen oder den von Triarylmethan abgeleiteten Farbstoffen oder deren Gemischen ausgewählt sind.

12. Verfahren nach einem der vorhergehenden Ansprüche, **dadurch gekennzeichnet, dass** die Zusammensetzung den zusätzlichen Direktfarbstoff in einem Mengenanteil von 0,0005 bis 12 Gew.-% und vorzugsweise 0,005 bis 6 Gew.-%, bezogen auf das Gesamtgewicht der Zusammensetzung, enthält.

13. Verfahren nach einem der vorhergehenden Ansprüche, **dadurch gekennzeichnet, dass** die Zusammensetzung ferner mindestens einen nichtionischen, anionischen, kationischen, amphoteren oder zwitterionischen grenzflächenaktiven Stoff oder deren Gemische enthält.

14. Verfahren nach dem vorhergehenden Anspruch, **dadurch gekennzeichnet, dass** die Zusammensetzung einen Tensidgehalt von 0,01 bis 40 Gew.-%, und vorzugsweise 0,1 bis 30 Gew.-%, bezogen auf das Gesamtgewicht der Zusammensetzung, aufweist.

15. Verfahren nach einem der vorhergehenden Ansprüche, **dadurch gekennzeichnet, dass** das kosmetisch akzeptable Medium Wasser und gegebenenfalls ein organisches Lösungsmittel enthält.

16. Verfahren nach einem der vorhergehenden Ansprüche, **dadurch gekennzeichnet, dass** die Zusammensetzung in Form eines färbenden Haarwaschmittels vorliegt.

17. Verfahren nach einem der Ansprüche 1 bis 15, **dadurch gekennzeichnet, dass** die Zusammensetzung in Form einer Mascara für die Wimpern oder Mascara für die Haare vorliegt.

18. Verfahren nach einem der Ansprüche 1 bis 15, **dadurch gekennzeichnet, dass** die Zusammensetzung mindestens eine Oxidationsbase enthält, die unter den *p*-Phenylendiaminen, Bisphenylalkylendiaminen, *p*-Aminophenolen, o-Aminophenolen und den heterocyclischen Basen oder deren Additionssalzen mit einer Säure oder mit einem alkalischen Stoff ausgewählt ist.

19. Verfahren nach einem der Ansprüche 1 bis 15 und 18, **dadurch gekennzeichnet, dass** die Zusammensetzung einen Mengenanteil der Oxidationsbase von 0,0005 bis 12 Gew.-% und vorzugsweise 0,005 bis 6 Gew.-%, bezogen auf das Gesamtgewicht der Zusammensetzung, enthält.

20. Verfahren nach einem der Ansprüche 1 bis 15 und 18 oder 19, **dadurch gekennzeichnet, dass** die Zusammensetzung ferner mindestens einen Kuppler enthält, der unter den m-Phenylendiaminen, *m*-Aminophenolen, *m*-Dihydroxybenzolen und den heterocyclischen Kupplern oder deren Additionssalzen mit einer Säure oder mit einem alkalischen Stoff ausgewählt ist.

21. Verfahren nach einem der Ansprüche 1 bis 15 und 18 bis 20, **dadurch gekennzeichnet, dass** die Zusammensetzung einen Mengenanteil des Kupplers von 0,0001 bis 10 Gew.-% und vorzugsweise 0,005 bis 5 Gew.-%, bezogen auf das Gesamtgewicht der Zusammensetzung, enthält.

22. Verfahren nach einem der Ansprüche 1 bis 15 und 18 bis 21, **dadurch gekennzeichnet, dass** die Zusammensetzung mindestens ein Oxidationsmittel enthält.

23. Verfahren nach dem vorhergehenden Anspruch, **dadurch gekennzeichnet, dass** das Oxidationsmittel unter Wasserstoffperoxid, Harnstoffperoxid, Alkalimetallbromaten, Salzen von Persäuren, wie Perboraten und Persulfaten, und Enzymen, beispielsweise Peroxidasen und Oxidoreduktasen (zwei oder vier Elektronen) ausgewählt ist.

24. Verfahren nach einem der Ansprüche 1 bis 15 und 17, **dadurch gekennzeichnet, dass** die folgenden Schritte durchgeführt werden:
a) die Zusammensetzung wird auf die menschlichen Keratinsubstanzen aufgebracht,
b) die Substanzen werden getrocknet oder trocknen gelassen.

25. Verfahren nach einem der Ansprüche 1 bis 16 und 18 bis 32, **dadurch gekennzeichnet, dass** die folgenden Schritte durchgeführt werden:
a) die Zusammensetzung wird während einer Zeitspanne, die für die Bildung der gewünschten Färbung und Aufhellung ausreichend ist, auf die menschlichen Keratinsubstanzen aufgebracht,
b) die so behandelten Substanzen werden gegebenenfalls gespült,
c) die Substanzen werden gegebenenfalls gewaschen und gespült,
d) die Substanzen werden getrocknet oder trocknen gelassen.

26. Fluoreszierende Verbindung der folgenden Formel: 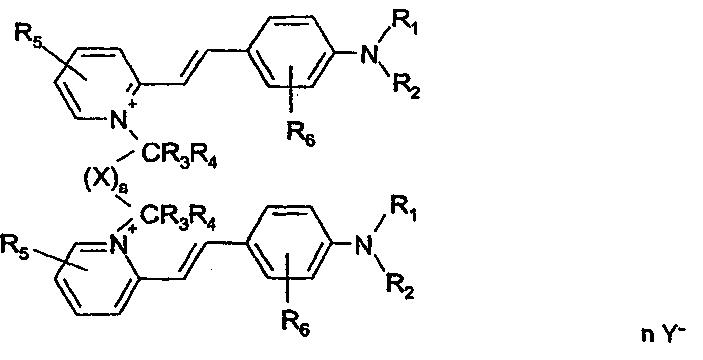 worin bedeuten:
die Gruppen R₁ und R₂, die gleich oder verschieden sind:
· ein Wasserstoffatom;
· eine geradkettige oder verzweigte Alkylgruppe mit 1 bis 10 Kohlenstoffatomen, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
· eine Aryl- oder Arylalkylgruppe, wobei die Arylgruppe 6 Kohlenstoffatome und die Alkylgruppe 1 bis 4 Kohlenstoffatome besitzt; wobei die Arylgruppe gegebenenfalls mit einer oder mehreren, geradkettigen oder verzweigten Alkylgruppen mit 1 bis 4 Kohlenstoffatomen substituiert ist, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert sind und/oder mit mindestens einem Halogenatom substituiert sind;
· die Gruppen R₁ und R₂ können gegebenenfalls so verbunden sein, dass sie mit dem Stickstoffatom einen Heterocyclus bilden, wobei ein oder mehrere weitere Heteroatome enthalten sein können, wobei der Heterocyclus gegebenenfalls mit mindestens einer geradkettigen oder verzweigten Alkylgruppe substituiert ist, die vorzugsweise 1 bis 4 Kohlenstoffatome enthält und gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
· die Gruppe R₁ oder R₂ kann gegebenenfalls in einen Heterocyclus eingebunden sein, der das Stickstoffatom und ein Kohlenstoffatom des Phenylrings, der das Stickstoffatom trägt, enthält.
die Gruppen R₃ und R₄, die identisch oder verschieden sind, ein Wasserstoffatom oder eine Alkylgruppe mit 1 bis 4 Kohlenstoff atomen;
die Gruppen R₅, die gleich oder verschieden sind, ein Wasserstoffatom; ein Halogenatom; eine geradkettige oder verzweigte Alkylgruppe mit 1 bis 4 Kohlenstoffatomen, die gegebenenfalls durch mindestens ein Heteroatom unterbrochen ist;
die Gruppen R₆, die gleich oder verschieden sind, ein Wasserstoffatom; ein Halogenatom; eine geradkettige oder verzweigte Alkylgruppe mit 1 bis 4 Kohlenstoffatomen, die gegebenenfalls mit mindestens einem Heteroatom und/oder einer Gruppe, die mindestens ein Heteroatom enthält, substituiert und/oder durch mindestens ein Heteroatom und/oder eine Gruppe, die mindestens ein Heteroatom enthält, unterbrochen ist und/oder mit mindestens einem Halogenatom substituiert ist;
X bedeutet:
· eine geradkettige oder verzweigte Alkylgruppe mit 1 bis 14 Kohlenstoffatomen oder eine Alkenylgruppe mit 2 bis 14 Kohlenstoffatomen, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert und/oder mit mindestens einem Halogenatom substituiert sind;
· eine 5- oder 6-gliedrige heterocyclische Gruppe, die gegebenenfalls mit mindestens einer geradkettigen oder verzweigten Alkylgruppe mit 1 bis 14 Kohlenstoffatomen, die gegebenenfalls mit mindestens einem Heteroatom substituiert ist; mit mindestens einer geradkettigen oder verzweigten Aminoalkylgruppe mit 1 bis 4 Kohlenstoffatomen, die gegebenenfalls mit mindestens einem Heteroatom substituiert ist; mit mindestens einem Halogenatom substituiert ist;
· eine aromatische Gruppe oder eine kondensierte oder nicht kondensierte, diaromatische Gruppe, die gegebenenfalls über eine Alkylgruppe mit 1 bis 4 Kohlenstoffatomen getrennt ist, wobei die Arylgruppe(n) gegebenenfalls mit mindestens einem Halogenatom oder mit mindestens einer Alkylgruppe mit 1 bis 10 Kohlenstoffatomen substituiert sind, die gegebenenfalls mit mindestens einem Heteroatom und/oder einer Gruppe, die mindestens ein Heteroatom enthält, substituiert und/oder durch mindestens ein Heteroatom und/oder eine Gruppe, die mindestens ein Heteroatom enthält, unterbrochen ist;
· eine Dicarbonylgruppe;
· wobei die Gruppe X eine oder mehrere kationische Ladungen umfassen kann,
a 0 oder 1;
die Gruppen Y⁻, die gleich oder verschieden sind, ein organisches oder anorganisches Anion;
wobei n eine ganze Zahl ist, die mindestens 2 und höchstens die Anzahl der in der fluoreszierenden Verbindung vorhandenen kationischen Ladungen bedeutet;
wobei Verbindungen ausgenommen sind, worin:
· die Gruppe X eine lineare, unsubstituierte Alkylgruppe mit 1 oder 4 Kohlenstoffatomen mit a = 1 oder a = 0 bedeutet; und die Gruppen R₁ und R₂ gleichzeitig eine Methylgruppe bedeuten, R₅ und R₆ ein Wasserstoffatom bedeuten; die Gruppen R₃ und R₄, die gleich sind, ein Wasserstoffatom bedeuten;
· die Gruppe X eine unsubstituierte, lineare C₂-Alkylgruppe, eine lineare oder verzweigte, unsubstituierte C₃-Alkylgruppe ist; a 1 bedeutet; R₃ und R₄, die gleich sind, ein Wasserstoffatom bedeuten; R₅ ein Wasserstoffatom ist; die Gruppen R₁ und R₂:
- identisch sind und eine Methylgruppe bedeuten, R₆ ein Wasserstoffatom ist oder eine Methylgruppe in ortho-Stellung bezogen auf das Kohlenstoffatom des Benzolrings mit der ungesättigten Kohlenstoff-Kohlenstoff-Bindung; oder die Gruppen R₁ und R₂ identisch sind und eine Ethylgruppe bedeuten, R₆ ein Wasserstoffatom ist oder eine Methoxygruppe in ortho-Stellung bezogen auf das Kohlenstoffatom des Benzolrings, mit der ungesättigten Kohlenstoff Kohlenstoff Bindung;
- voneinander verschieden sind und eine Ethylgruppe und eine mit einer Dimethylaminogruppe, Trimethylammoniumgruppe, Benzyldimethylammoniumgruppe substituierte Ethylgruppe bedeuten;
· die Gruppe X eine Phenylgruppe bedeutet, die Bindungen zu den Gruppen CR₃R₄ in 1,4-Stellung zueinander aufweist; die Gruppen R₃ und R₄, die gleich sind, ein Wasserstoffatom bedeuten; a 1 ist; R₅ ein Wasserstoffatom bedeutet; die Gruppen R₁ und R₂:
- identisch sind und eine Methylgruppe bedeuten, R₆ ein Wasserstoffatom ist oder eine Methylgruppe in ortho-Stellung bezogen auf das Kohlenstoffatom des Benzolrings mit der ungesättigten Kohlenstoff-Kohlenstoff-Bindung; oder die Gruppen R₁ und R₂ identisch sind und eine Ethylgruppe bedeuten, R₆ ein Wasserstoffatom ist oder eine Methoxygruppe in ortho-Stellung bezogen auf das Kohlenstoffatom des Benzolrings mit der ungesättigten Kohlenstoff-Kohlenstoff-Bindung;
- voneinander verschieden sind und eine Ethylgruppe und eine mit einer Dimethylaminogruppe, Trimethylammoniumgruppe, Benzyldimethylammoniumgruppe substituierte Ethylgruppe bedeuten.

27. Verbindung nach Anspruch 26, **dadurch gekennzeichnet, dass** die Gruppen R₁ und R₂, die gleich oder verschieden sind, bedeuten:
· ein Wasserstoffatom;
· eine Alkylgruppe mit 1 bis 6 Kohlenstoffatomen, die gegebenenfalls durch ein Sauerstoffatom unterbrochen oder gegebenenfalls mit mindestens einer Hydroxygruppe, Aminogruppe, Ammoniumgruppe, einem Chloratom oder einem Fluoratom substituiert ist;
· eine Benzylgruppe, eine Phenylgruppe, die gegebenenfalls mit einer Alkyl- oder Alkoxygruppe mit 1 bis 4 Kohlenstoffatomen und vorzugsweise 1 oder 2 Kohlenstoffatomen substituiert sind;
· mit dem Stickstoffatom eine heterocyclische Gruppe vom Typ Pyrrolo, Pyrrolidino, Imidazolino, Imidazolo, Imidazolium, Pyrazolino, Piperazino, Morpholino, Morpholo, Pyrazolo, Triazolo, die gegebenenfalls mit mindestens einer geradkettigen oder verzweigten Alkylgruppe mit 1 bis 4 Kohlenstoffatomen substituiert ist, die gegebenenfalls durch ein Stickstoff- und/ oder Sauerstoffatom und/oder eine Gruppe, die ein Stickstoff- und/oder Sauerstoffatom trägt, unterbrochen und/oder mit einem Stickstoff- und/oder Sauerstoffatom und/oder einer Gruppe substituiert ist, die ein Stickstoff- und/oder Sauerstoffatom trägt.

28. Verbindung nach einem der Ansprüche 26 oder 27, **dadurch gekennzeichnet, dass** die Gruppen R₁ und R₂, die gleich oder verschieden sind, eine Alkylgruppe mit 1 bis 4 Kohlenstoffatomen bedeuten.

29. Verbindung nach dem vorhergehenden Anspruch, **dadurch gekennzeichnet, dass** die Gruppen R₁ und R₂, die gleich oder verschieden sind, Methyl, Ethyl bedeuten.

30. Verbindung nach einem der Ansprüche 26 bis 29, **dadurch gekennzeichnet, dass** die Gruppen R₅ und R₆ ein Wasserstoffatom bedeuten.

31. Verbindung nach einem der Ansprüche 26 bis 30, **dadurch gekennzeichnet, dass** X bedeutet:
· eine verzweigte Alkylgruppe mit 4 bis 14 Kohlenstoffatomen; eine geradkettige oder verzweigte Alkylgruppe mit 1 bis 14 Kohlenstoffatomen, die mit einem oder mehreren Halogenatomen und/oder Heteroatomen und/oder Gruppen, die ein oder mehrere Heteroatome enthalten, substituiert ist; eine geradkettige oder verzweigte Alkylgruppe mit 1 bis 14 Kohlenstoffatomen, die durch ein oder mehrere Heteroatome und/oder Gruppen, die ein oder mehrere Heteroatome enthalten, unterbrochen ist; eine geradkettige oder verzweigte Alkengruppe mit 2 bis 14 Kohlenstoffatomen, die gegebenenfalls durch ein oder mehrere Heteroatome oder Gruppen, die ein oder mehrere Heteroatome enthalten, unterbrochen und/oder mit einem oder mehreren Heteroatomen oder Gruppen, die ein oder mehrere Heteroatome enthalten, substituiert ist;
· eine Dicarbonylgruppe;
· eine 5- oder 6-gliedrige heterocyclische Gruppe, die gegebenenfalls mit mindestens einer Alkylgruppe mit 1 bis 14 Kohlenstoffatomen, die gegebenenfalls mit mindestens einem Heteroatom substituiert ist; mit mindestens einer geradkettigen oder verzweigten Aminoalkylgruppe mit 1 bis 4 Kohlenstoffatomen, die gegebenenfalls mit mindestens einem Heteroatom substituiert ist; mit mindestens einem Halogenatom substituiert ist;
·eine aromatische C₆-Gruppe, die an die Gruppen CR₃R₄ über Bindungen in 1,3-Stellung zueinander gebunden ist, gegebenenfalls mit einem oder mehreren Halogenatomen und/oder C₁-4-Alkylgruppen substituiert ist; wobei die Bindungen gleiche oder verschiedene, lineare oder verzweigte C₁₋₄-Gruppen umfassen und eine Gruppe aufweisen, die ein oder mehrere Heteroatome enthält und direkt oder indirekt an die aromatische Gruppe gebunden ist; eine aromatische C₆-Gruppe, die an die Gruppen CR₃R₄ über Bindungen in 1,2-Stellung zueinander gebunden ist, wobei die Gruppen eine identische oder nicht identische, lineare oder verzweigte C₁-₄-Alkylgruppe umfassen und eine Gruppe aufweisen, die ein oder mehrere Heteroatome enthält und direkt oder indirekt an die aromatische Gruppe gebunden ist; eine aromatische C₆-Gruppe, die an die Gruppen CR₃R₄ über Bindungen in 1,2-Stellung zueinander gebunden ist, mit einer oder mehreren Gruppen substituiert ist, die ein oder mehrere Heteroatome aufweisen; eine aromatische C₆-Gruppe, die an die Gruppen CR₃R₄ über Bindungen in 1,4-Stellung zueinander gebunden ist, wobei die Bindungen eine identische oder nicht identische, lineare oder verzweigte C₁₋₄-Alkylgruppe umfassen und eine Gruppe aufweisen, die ein oder mehrere Heteroatome enthält und direkt oder nicht direkt an die aromatische Gruppe gebunden ist; eine Diphenylgruppe, die an die Gruppen CR₃R₄ über Bindungen in 4,4'-Stellung zueinander gebunden ist, wobei die beiden aromatischen Kerne gegebenenfalls über eine geradkettige oder verzweigte C₁₋₄-Alkylgruppe verbunden sind;
. wobei die Gruppe X eine oder mehrere kationische Ladungen aufweisen kann.

32. Verbindung nach einem der Ansprüche 26 bis 31, **dadurch gekennzeichnet, dass** X bedeutet:
· eine verzweigte Alkylgruppe mit 4 bis 13 Kohlenstoffatomen; eine geradkettige oder verzweigte Alkylgruppe mit 1 bis 13 Kohlenstoffatomen, die mit mindestens einem Chloratom und/oder mindestens einer Hydroxygruppe und/oder mindestens einer Acetoxygruppe und/oder mindestens einer Aminogruppe und/oder mindestens einer Ammoniumgruppe substituiert ist; eine geradkettige oder verzweigte Alkylgruppe mit 2 bis 12 Kohlenstoffatomen, die durch ein oder mehrere Sauerstoffatome, ein oder mehrere Stickstoffatome unterbrochen ist, die eine oder zwei Gruppen tragen, die gleich oder verschieden sind und unabhängig voneinander unter Wasserstoffatomen, geradkettigen oder verzweigten C₁₋₄-Alkylgruppen, die gegebenenfalls eine Hydroxygruppe aufweisen, unterbrochen sind; eine geradkettige oder verzweigte Alkengruppe mit 2 bis 12 Kohlenstoffatomen, die eine ungesättigte Kohlenstoff-Kohlenstoff-Bindung aufweist;
· eine Pyridingruppe, die an die Gruppen CR₃R₄ über Bindungen in 2- und 6-Stellung zueinander gebunden ist;
· eine Imidazolgruppe, die gegebenenfalls mit mindestens einer C₁₋₁₄-Alkylgruppe substituiert ist;
· eine aromatische C₆-Gruppe, die an die Gruppen CR₃R₄ über Bindungen in 1,3-Stellung zueinander gebunden ist, die gegebenenfalls mit einem oder mehreren Fluoratomen und/oder Methyl substituiert ist; wobei diese Bindungen eine identische oder nicht identische, lineare oder verzweigte C₁-₄-Alkylgruppe umfassen und eine Gruppe aufweisen, die ein oder mehrere Heteroatome enthält und direkt oder indirekt an die aromatische Gruppe gebunden ist; eine aromatische C₆-Gruppe, die an die Gruppen CR₃R₄ über Bindungen in 1,2-Stellung zueinander gebunden ist, wobei die Bindungen eine identische oder nicht identische, lineare oder verzweigte C₁₋₄-Alkylgruppe umfassen und eine Gruppe aufweisen, die ein Sauerstoffatom aufweist und direkt an die aromatische Gruppe gebunden ist; eine aromatische C₆-Gruppe, die an die Gruppen CR₃R₄ über Bindungen in 1,2-Stellung zueinander gebunden ist, die mit einer oder mehreren Gruppen vom Typ -C(=O)O- substituiert ist; eine aromatische C₆-Gruppe, die an die Gruppen CR₃R₄ über Bindungen in 1,4-Stellung zueinander gebunden ist, wobei die Bindungen eine identische oder nicht identische, lineare oder verzweigte C₁₋₄-Alkylgruppe umfassen und eine direkt an die aromatische Gruppe gebundene Amidgruppe aufweisen; eine Diphenylgruppe, die an die Gruppen CR₃R₄ über Bindungen in 4,4'-Stellung zueinander gebunden ist, wobei die beiden aromatischen Kerne gegebenenfalls über eine geradkettige oder verzweigte Alkylgruppe mit 1 bis 4 Kohlenstoffatomen und vorzugsweise 1 oder 2 Kohlenstoffatomen verbunden sind;
· wobei die Gruppe X eine oder mehrere kationische Ladungen aufweisen kann.

33. Verbindung nach einem der Ansprüche 26 bis 31, **dadurch gekennzeichnet, dass** X bedeutet:
· eine verzweigte Alkylgruppe mit 4 bis 13 Kohlenstoffatomen; eine geradkettige oder verzweigte Alkylgruppe mit 1 bis 13 Kohlenstoffatomen, die gegebenenfalls mit mindestens einem Chloratom und/oder mindestens einer Hydroxygruppe und/oder mindestens einer Acetoxygruppe und/oder mindestens einer Aminogruppe und/oder mindestens einer Ammoniumgruppe substituiert ist; eine geradkettige oder verzweigte Alkylgruppe mit 2 bis 12 Kohlenstoffatomen, die durch ein oder mehrere Sauerstoffatome, ein oder mehrer Stickstoffatome unterbrochen ist, die ein oder zwei Gruppen tragen, die gleich oder verschieden und unabhängig voneinander unter Wasserstoff, geradkettigen oder verzweigten C₁₋₄-Alkylgruppen, die gegebenenfalls eine Hydroxygruppe tragen, ausgewählt sind; eine geradkettige oder verzweigte Alkengruppe mit 2 bis 12 Kohlenstoffatomen, die eine ungesättigte Kohlenstoff-Kohlenstoff-Bindung aufweist;
· eine Imidazolgruppe, die gegebenenfalls mit mindestens einer C₁₋₁₄-Alkylgruppe substituiert ist;
· eine aromatische C₆-Gruppe, die an die Gruppen CR₃R₄ über Bindungen in 1,3-Stellung zueinander gebunden ist, die gegebenenfalls mit einem oder mehreren Fluoratomen und/oder Methyl substituiert ist; wobei die Bindungen eine identisch oder nicht identische, lineare oder verzweigte C₁₋₄-Alkylgruppe umfassen und eine Gruppe aufweisen, die ein oder mehrere Heteroatome enthält und direkt oder indirekt an die aromatische Gruppe gebunden ist; eine aromatische C₆-Gruppe, die an die Gruppen CR₃R₄ über Bindungen in 1,4-Stellung zueinander gebunden ist, wobei die Bindungen eine identische oder nicht identische, geradkettige oder verzweigte C₁₋₄-Alkylgruppe umfassen und eine direkt an die aromatische Gruppe gebundene Amidgruppe aufweisen;
· wobei die Gruppe X eine oder mehrere kationischen Ladungen aufweisen kann.

34. Verbindung nach einem der Ansprüche 26 bis 31, **dadurch gekennzeichnet, dass**:
Die Gruppen R₁ und R₂, die identisch oder nicht identisch sind, eine Methylgruppe, eine Ethylgruppe bedeuten;
die Gruppen R₅ und R₆ ein Wasserstoffatom bedeuten;
X bedeutet:
· eine verzweigte Alkylgruppe mit 4 bis 13 Kohlenstoffatomen; eine geradkettige oder verzweigte Alkylgruppe mit 1 bis 13 Kohlenstoffatomen, die gegebenenfalls mit mindestens einem Chloratom und/oder mindestens einer Hydroxygruppe und/oder mindestens einer Acetoxygruppe und/oder mindestens einer Aminogruppe und/oder mindestens einer Ammoniumgruppe substituiert ist; eine geradkettige oder verzweigte Alkylgruppe mit 2 bis 12 Kohlenstoffatomen, die durch ein oder mehrere Sauerstoffatome, ein oder mehrer Stickstoffatome unterbrochen ist, die eine oder zwei Gruppen tragen, die gleich oder verschieden und unabhängig voneinander unter Wasserstoff, geradkettigen oder verzweigten C₁₋₄-Alkylgruppen, die gegebenenfalls eine Hydroxygruppe tragen, ausgewählt sind; eine geradkettige oder verzweigte Alkengruppe mit 2 bis 12 Kohlenstoffatomen, die eine ungesättigte Kohlenstoff-Kohlenstoff-Bindung aufweist;
· eine Imidazolgruppe, die gegebenenfalls mit mindestens einer C₁₋₁₄-Alkylgruppe substituiert ist;
· eine aromatische C₆-Gruppe, die an die Gruppen CR₃R₄ über Bindungen in 1,3-Stellung zueinander gebunden ist und die gegebenenfalls mit einem oder mehreren Fluoratomen und/oder Methyl substituiert ist; wobei die Bindungen eine identische oder nicht identische, lineare oder verzweigte C₁₋₄-Alkylgruppe umfassen und eine Gruppe aufweisen, die ein oder mehrere Heteroatome enthält und direkt oder indirekt an die aromatische Gruppe gebunden ist; eine aromatische C₆-Gruppe, die an die Gruppen CR₃R₄ über Bindungen in 1,4-Stellung zueinander gebunden ist, wobei die Bindungen eine identische oder nicht identische, geradkettige oder verzweigte C₁₋₄-Alkylgruppe umfassen und eine direkt an die aromatische Gruppe gebundene Amidgruppe aufweisen;
· wobei die Gruppe X eine oder mehrere kationischen Ladungen aufweisen kann.

35. Zusammensetzung, die in einem kosmetisch akzeptablen Medium mindestens eine fluoreszierende Verbindung nach einem der Ansprüche 26 bis 35 enthält.

36. Zusammensetzung nach dem vorhergehenden Anspruch, **dadurch gekennzeichnet, dass** der Mengenanteil der fluoreszierenden Verbindung im Bereich von 0,01 bis 20 Gew.-%, insbesondere 0,05 bis 10 Gew.-% und vorzugsweise 0,1 bis 5 Gew.-%, bezogen auf das Gesamtgewicht der Zusammensetzung, liegt.

37. Zusammensetzung nach einem der Ansprüche 35 oder 36, **dadurch gekennzeichnet, dass** die Zusammensetzung mindestens einen ergänzenden, nicht fluoreszierenden Direktfarbstoff enthält.

38. Zusammensetzung nach dem vorhergehenden Anspruch, **dadurch gekennzeichnet, dass** der oder die zusätzlichen Direktfarbstoffe unter den nitrierten Benzolfarbstoffen, Azofarbstoffen, Azomethin-Farbstoffen, Methin-Farbstoffen, Anthrachinon-Farbstoffen, Naphthochinon-Farbstoffen, Benzochinon-Farbstoffen, Phenothiazin-Farbstoffen, Indigoiden, Xanthen-Farbstoffen, Phenanthridin-Farbstoffen, Phthalocyaninen, von Triarylmethan abgeleiteten Farbstoffen oder deren Gemischen ausgewählt sind.

39. Zusammensetzung nach einem der Ansprüche 35 bis 38, **dadurch gekennzeichnet, dass** der Mengenanteil des zusätzlichen Direktfarbstoffs im Bereich on 0,0005 bis 12 Gew.-% und vorzugsweise 0,005 bis 6 Gew.-%, bezogen auf das Gesamtgewicht der Zusammensetzung, liegt.

40. Zusammensetzung nach einem der Ansprüche 35 bis 39, **dadurch gekennzeichnet, dass** die Zusammensetzung mindestens einen nichtionischen, anionischen, kationischen, amphoteren oder zwitterionischen grenzflächenaktiven Stoff oder deren Gemische enthält.

41. Zusammensetzung nach dem vorhergehenden Anspruch, **dadurch gekennzeichnet, dass** der Tensidgehalt im Bereich von 0,01 bis 40 Gew.-% und vorzugsweise 0,1 bis 30 Gew.-%, bezogen auf das Gesamtgewicht der Zusammensetzung, liegt.

42. Zusammensetzung nach einem der Ansprüche 35 bis 41, **dadurch gekennzeichnet, dass** das kosmetisch akzeptable Medium Wasser und gegebenenfalls ein organisches Lösungsmittel umfasst.

43. Zusammensetzung nach einem der Ansprüche 35 bis 42, **dadurch gekennzeichnet, dass** sie als färbendes Haarwaschmittel vorliegt.

44. Zusammensetzung nach einem der Ansprüche 35 bis 43, **dadurch gekennzeichnet, dass** sie als Mascara für die Wimpern oder Mascara für die Haare vorliegt.

45. Zusammensetzung nach einem der Ansprüche 35 bis 44, **dadurch gekennzeichnet, dass** die Zusammensetzung ferner mindestens eine Oxidationsbase enthält, die unter den *p*-Phenylendiaminen, Bisphenylalkylendiaminen, p-Aminophenolen, o-Aminophenolen und heterocyclischen Basen oder deren Additionssalzen mit einer Säure oder einem alkalischen Stoff ausgewählt ist.

46. Zusammensetzung nach einem der Ansprüche 35 bis 42 und 45, **dadurch gekennzeichnet, dass** der Mengenanteil der Oxidationsbase im Bereich von 0,0005 bis 12 Gew.-% und vorzugsweise 0,005 bis 6 Gew.-%, bezogen auf das Gesamtgewicht der Zusammensetzung, liegt.

47. Zusammensetzung nach einem der Ansprüche 35 bis 42 und 45 oder 46, **dadurch gekennzeichnet, dass** sie ferner mindestens einen Kuppler enthält, der unter den m-Phenylendiaminen, m-Aminophenolen, *m*-Dihydroxybenzolen und den heterocyclischen Kupplern oder deren Additionssalzen mit einer Säure oder einem alkalischen Stoff ausgewählt ist.

48. Zusammensetzung nach einem der Ansprüche 35 bis 42 und 45 bis 47, **dadurch gekennzeichnet, dass** sie einen Mengenanteil des Kupplers im Bereich von 0,0001 bis 10 Gew.-% und vorzugsweise 0,005 bis 5 Gew.-%, bezogen auf das Gesamtgewicht der Zusammensetzung, enthält.

49. Zusammensetzung nach einem der Ansprüche 35 bis 42 und 45 bis 48, **dadurch gekennzeichnet, dass** die Zusammensetzung ferner mindestens ein Oxidationsmittel enthält.

50. Zusammensetzung nach dem vorhergehenden Anspruch, **dadurch gekennzeichnet, dass** das Oxidationsmittel unter Wasserstoffperoxid, Harnstoffperoxid, Alkalimetallbromaten, Salzen von Persäuren wie Perboraten und Persulfaten und Enzymen, wie Peroxidasen und Oxidoreduktasen (2 oder 4 Elektronen) ausgewählt ist.

51. Vorrichtung mit mehreren Abteilungen, die mindestens eine Abteilung mit einer Zusammensetzung nach einem der Ansprüche 35 bis 42 und 45 bis 47, die in einem kosmetisch akzeptablen Medium mindestens eine spezielle fluoreszierende Verbindung und gegebenenfalls mindestens einen zusätzlichen Direktfarbstoff und/oder mindestens eine Oxidationsbase und/oder mindestens einen Kuppler enthält, und mindestens eine weitere Abteilung aufweist, die eine Zusammensetzung mit mindestens einem Oxidationsmittel enthält.
